(19) Europäisches Patentamt
European Patent Office
Office européen des brevets

(11) EP 1 977 646 A1

(12) **EUROPEAN PATENT APPLICATION**

(43) Date of publication:
**08.10.2008 Bulletin 2008/41**

(21) Application number: **07006900.0**

(22) Date of filing: **03.04.2007**

(51) Int Cl.:
***A01N 47/32*** *(2006.01)*
***A01N 47/36*** *(2006.01)*
***C07C 331/26*** *(2006.01)*
***C07C 335/14*** *(2006.01)*
***C07D 307/82*** *(2006.01)*
***C07D 311/68*** *(2006.01)*
***C07D 335/06*** *(2006.01)*
***A01P 7/02*** *(2006.01)*
***A01P 7/04*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Bayer CropScience AG**
**40789 Monheim (DE)**

(72) Inventors:
- **Schwarz, Hans-Georg, Dr.**
**40764 Langenfeld (DE)**
- **Andree, Roland, Dr.**
**40764 Langenfeld (DE)**
- **Holmwood, Graham, Dr.**
**51371 Leverkusen (DE)**
- **Schallner, Otto, Dr.**
**40789 Monheim (DE)**
- **Antonicek, Horst-Peter, Dr.**
**51467 Bergisch Gladbach (DE)**
- **Franken, Eva-Maria, Dr.**
**42799 Leichlingen (DE)**
- **Malsam, Olga, Dr.**
**51503 Rösrath (DE)**
- **Arnold, Christianf, Dr.**
**40764 Langenfeld (DE)**
- **Görgens, Ulrich**
**40882 Ratingen (DE)**

(54) **Insecticidal hydroxyethyl thlourea derivatives**

(57) The present invention relates to novel derivatives of substituted hydroxyethyl thiourea derivatives, to processes for their preparation and to their use for controlling animal pests, especially arthropods, in particular insects.

EP 1 977 646 A1

## Description

The present application relates to novel hydroxyethyl thiourea derivatives, to processes for their preparation and to their use for controlling animal pests, especially arthropods, in particular insects.

Certain hydroxyethyl thioureas compounds are already known as insecticidally active compounds from WO 2006/125745. Certain 2-hydroxethyl-thioureas are known from WO 2007/020377 as intermediates, but no biological activity is reported.

Since modern pesticides must meet a wide range of demands, for example regarding level, duration and spectrum of action, use spectrum, toxicity, combination with other active substances, combination with formulation auxiliaries or synthesis, and since the occurrence of resistances is possible, the development of such substances can never be regarded as concluded, and there is constantly a high demand for novel compounds which are advantageous over the known compounds, at least as far as some aspects are concerned.

This invention now provides novel compounds of formula (I)

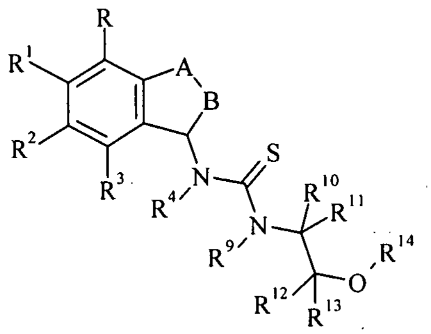

(I)

wherein

A is selected from $-CHR^5-$, $-CHR^5CHR^6-$, -O-, $-S(O)_n-$, $-NR^7-$, $-OCHR^5-$, $-CHR^5O-$, $-S(O)_nCHR^5-$, $-CHR^5S(O)_n-$, -C(X)- or $-C(=NOR^7)-$;

B is selected from $-CHR^8-$, -O- and $-S(O)_n-$;

X is selected from oxygen and sulphur;

n is an integer selected from 0, 1 or 2;

R, $R^1$, $R^2$ and $R^3$ are independently selected from hydrogen, halogen, hydroxy, alkyl, haloalkyl, alkoxy, haloalkoxy, alkylthio, haloalkylthio, alkenyl, haloalkenyl, alkynyl, cyano, nitro, optionally substituted aryl, optionally substituted aryloxy, optionally substituted heteroaryl, optionally substituted heteroaryloxy, alkoxyalkyl, alkoxyalkoxyalkyl, cycloalkyl, cyanoalkyl, formyl, alkoxycarbonyl, alkylcarbonyl, alkenylcarbonyl, dialkylphosphonato, amino, monoalkylamino, dialkylamino, cycloalkylamino, (cycloalkyl)(alkyl)amino, alkylsulfonyl, haloalkylsulfonyl, alkylsulfinyl, haloalkylsulfinyl, alkylsulfinylalkyl, alkylsulfonylalkyl, alkylthiocarbonyl and hydroxycarbonyl;

$R^4$ is selected from hydrogen, amino, hydroxyl, cyano, alkyl, haloalkyl, cycloalkyl, optionally substituted arylalkyl, alkenyl, alkynyl, cycloalkylalkyl, alkoxy, hydroxyalkyl, alkylsulfonyl, $-C(X)R^{15}$, $-C(X)OR^{16}$, $-C(X)SR^{16}$, $-S(O)_nR^{16}$, $-C(X)NR^{17}R^{18}$, $-S(O)_nNR^{17}R^{18}$, $-P(X)(OR^{19})(OR^{20})$, $-P(X)(NR^{21}R^{22})(NR^{23}R^{24})$, $-CHR^{25}-X-R^{26}$, $-CH=NR^{27}$ and $-C(=NR^{31})(SR^{32})$;

$R^5$ is selected from hydrogen, halogen, hydroxy, alkyl, alkoxy, alkylthio, haloalkyl, haloalkoxy, cyano, nitro and optionally substituted aryl;

$R^6$ is selected from hydrogen, halogen, hydroxy, alkyl, alkoxy, alkylthio, haloalkyl, haloalkoxy, cyano, nitro and optionally substituted aryl;

$R^7$ is selected from hydrogen or alkyl;

$R^8$ is selected from hydrogen, halogen, alkyl, alkoxy or optionally substituted aryl;

$R^9$ is selected from hydrogen, amino, hydroxyl, alkyl, haloalkyl, alkoxyalkyl, cycloalkyl, optionally substituted arylalkyl, alkenyl, alkynyl, cycloalkylalkyl, alkylcarbonyl, alkoxycarbonyl, alkylamino(thio)carbonyl, dialkylamino(thio)carbonyl, alkoxy, hydroxyalkyl;

$R^{10}$, $R^{11}$, $R^{12}$ and $R^{13}$ are independently selected from hydrogen, alkyl, haloalkyl, alkoxy, alkoxyalkyl, alkenyl, cycloalkyl, cycloalkylalkyl, optionally substituted aryl, optionally substituted arylalkyl, optionally substituted hetaryl and optionally substituted hetarylalkyl;

$R^{14}$ is selected from hydrogen, alkyl, haloalkyl, alkoxyalkyl, hydroxyalkyl, cycloalkyl, optionally substituted arylalkyl, alkenyl, alkynyl, cycloalkylalkyl, alkylcarbonyl, alkoxycarbonyl, alkylamino(thio)carbonyl, dialkylamino(thio)carbonyl, optionally substituted arylcarbonyl and optionally substituted aryloxycarbonyl,

$R^{15}$ is selected from hydrogen and alkyl;

$R^{16}$ is selected from alkyl, haloalkyl, hydroxyalkyl, hydroxycarbonylalkyl and alkoxycarbonylalkyl;

$R^{17}$ and $R^{18}$ are independently selected from hydrogen, alkyl, haloalkyl, cycloalkyl, cyanoalkyl, optionally substituted aryl, alkylcarbonyl, alkoxycarbonyl, alkoxycarbonylalkyl, alkoxyalkyl, alkylthioalkyl, alkylsulfinylalkyl, alkylsulfonylalkyl, mono or dialkylaminocarbonylalkyl, aminoalkyl, mono or dialkylaminoalkyl and optionally substituted arylcarbonyl;

$R^{19}$ and $R^{20}$ are independently selected from hydrogen, alkyl and haloalkyl;

$R^{21}$, $R^{22}$, $R^{23}$ and $R^{24}$ are independently selected from hydrogen and alkyl;

$R^{25}$ is selected from hydrogen and alkyl;

$R^{26}$ is selected from alkyl, haloalkyl, $-C(X)R^{28}$ and $-P(X)(OR^{29})(OR^{30})$;

$R^{27}$ is selected from hydrogen, alkyl, haloalkyl, hydroxy, alkoxy, haloalkoxy and cyano;

$R^{28}$ is selected from hydrogen, alkyl and haloalkyl;

$R^{29}$ and $R^{30}$ are independently selected from hydrogen, alkyl and haloalkyl;

$R^{31}$ and $R^{32}$ are independently selected from hydrogen, alkyl, arylalkyl, $-C(X)R^{15}$, $-CO_2R^{16}$, $S(O)_nR^{16}$, $-C(X)NR^{17}R^{18}$, $-S(O)_nNR^{17}R^{18}$, $-P(X)(OR^{19})(OR^{20})$, $-P(X)(NR^{21}R^{22})(NR^{23}R^{24})$ and $-CHR^{25}-X-R^{26}$;
and
agriculturally acceptable salts thereof.

One skilled in the art will, of course, recognize that within the description set forth above, when A is selected from -O-, $-S(O)_n-$, $-NR^8-$, $-CHR^6O-$ and $-CHR^6S(O)_n-$, then B is not selected from -O- or $-S(O)_n-$.

It has furthermore been found that the novel compounds of the formula (I) have pronounced biological properties and are suitable especially for controlling animal pests, in particular insects, arachnids and nematodes encountered in agriculture, in forests, in the protection of stored products and in the protection of materials, and also in the hygiene sector.

The present invention is also directed to compositions containing an insecticidally effective amount of at least one of a compound of formula I, and optionally, an effective amount of at least one of an additional compound, with at least one agriculturally acceptable extender or adjuvant.

The present invention is also directed to methods of controlling insects, where control is desired, which comprise applying an insecticidally effective amount of the above composition to the locus of crops, or other areas where insects are present or are expected to be present. Other aspects of the present invention will become apparent.

Furthermore, it has been found that the novel hydroxyethyl thiourea derivatives of the formula (I) can be obtained by the general processes provided in Schemes 1-3:

Scheme 1 (Process 1)

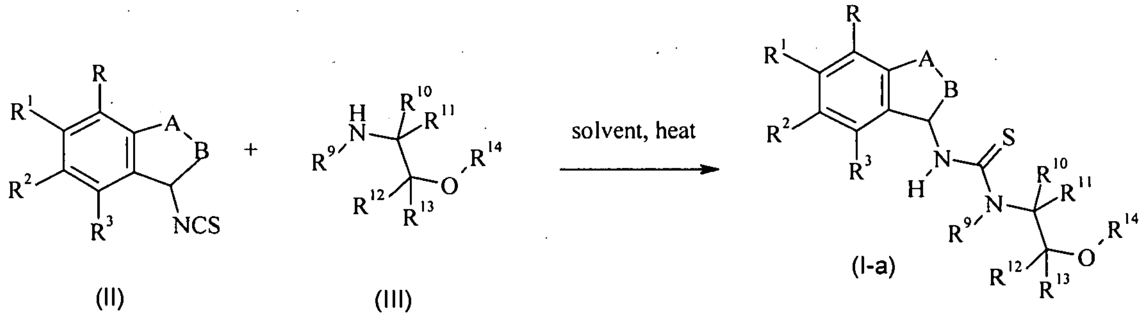


(II) (III) (I-a)

As depicted in Scheme 1, an appropriately substituted isothiocyanate (II) can be reacted with a optionally substituted aminoethanol (III) in a suitable solvent to yield the hydroxyethyl thiourea derivatives of formula (I-a) in which $R^4$ is hydrogen.

Scheme 2 (Process 2)

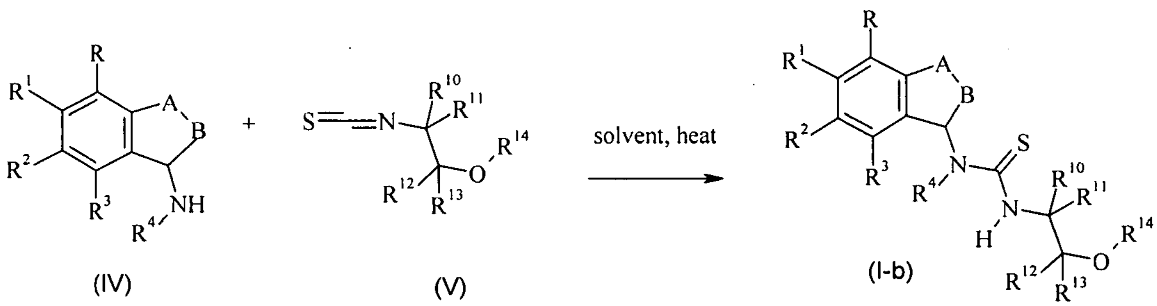


(IV) (V) (I-b)

As depicted in Scheme 2, an appropriately substituted amine (IV) can be reacted with an optionally substituted isothiocyanate (V) in a suitable solvent to yield the hydroxyethyl thiourea derivatives of formula (I-b) in which $R^9$ is hydrogen.

Scheme 3 (Process 3)

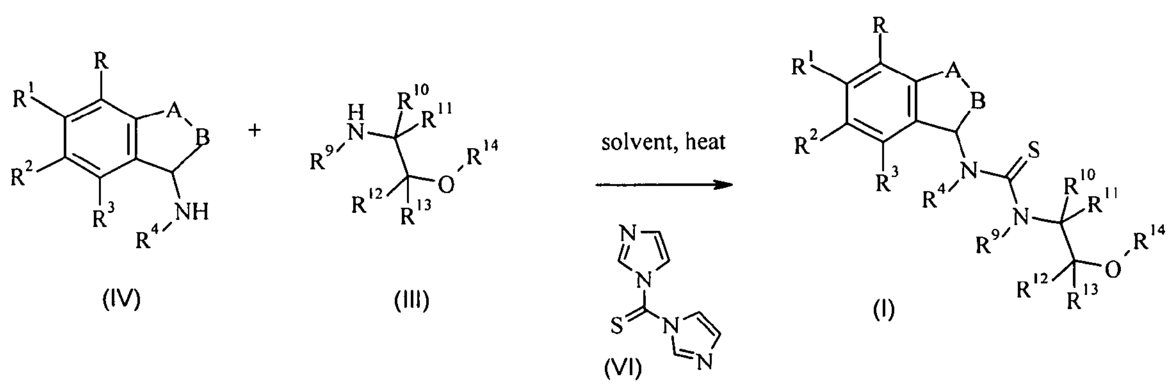


(IV) (III) (VI) (I)

As depicted in Scheme 3, an appropriately substituted amine (IV) can be reacted with an optionally substituted aminoethanol (III) in the presence of thiocarbonyl diimidazole (VI) in a suitable solvent to yield the hydroxyethyl thiourea

derivatives of formula (I).

Furthermore, this invention provides novel chiral intermediates of formula (II-a):

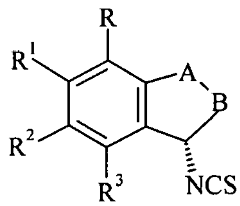

(II-a)

in which

A, B, R, $R^1$, $R^2$ and $R^3$ are as defined above, except the compound in which R = $R^1$ = $R^2$ = $R^3$ = hydrogen and A = $CH_2$ and B = $CH_2$, which is known from US200604019.

The relative stereochemistry is determined according to the so called Cahn-Ingold-Prelog priority rules and leads to (S) or (R) enantiomers, depending on the nature of the substituents which are involved.

It has been found, that isothiocyanates (II-a) can be obtained by the general processes shown in Scheme 4. The chiral amines (IV-a) are available according to known methods, cf. e.g. J. Org. Chem., 2006, 71(18), 6859 or Chem. Eur. J., 2000, 6(10), 1840.

Scheme 4 (Process 4)

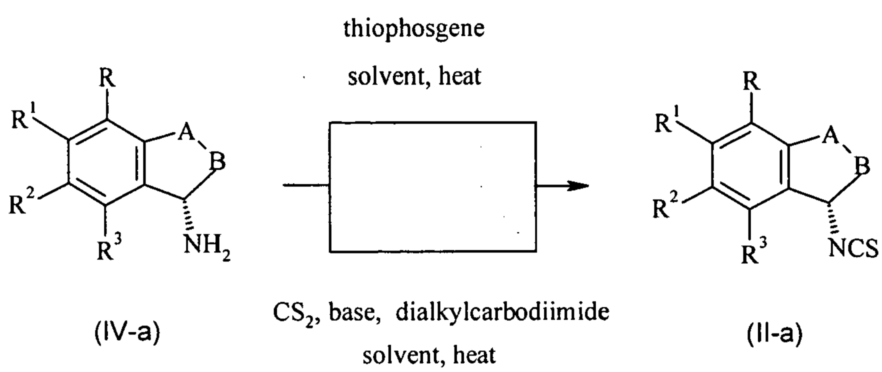


(IV-a) (II-a)

As depicted in Scheme 4, an appropriately substituted amine (IV-a) can be reacted with thiophosgene or carbon disulfide in the presence of a base and a dialkylcarbodiimide in a suitable solvent to yield the pure chiral isothiocyanates of formula (II-a).

The formula (I) provides a general definition of the compounds according to the invention.

The compounds of formula (I) may exist in different geometric or optical isomers or different tautomeric forms. Besides the C-atom adjacent to group B in the compounds of formula (I), one or more additional centres of chirality may be present. Therefore the compounds of the formula (I) may be present as pure enantiomers, mixtures of enantiomers, pure diastereomers or mixtures of diastereomers. There may be double bonds present in the molecule, such as C=C or C=N bonds, in which case compounds of formula (I) may exist as single isomers of mixtures of isomers. Centres of tautomerisation may be present. This invention covers all such isomers and tautomers and mixtures thereof in all proportions as well as isotopic forms such as deuterated compounds.

Preferred substituents or ranges of the radicals given in the formulae mentioned above and below are illustrated below.

Each alkyl moiety either alone or as part of a larger group (such as alkoxy, alkoxycarbonyl, alkylthio, alkylcarbonyl, alkylaminocarbonyl, dialkylaminocarbonyl) is a straight or branched chain and is, for example, methyl, ethyl, n-propyl, n-butyl, n-pentyl, n-hexyl, iso-propyl, n-butyl, sec-butyl, iso-butyl, tert-butyl or neo-pentyl. The alkyl groups are suitably $C_1$-$C_{12}$-alkyl groups, but are preferably $C_1$-$C_{10}$, more preferably $C_1$-$C_8$, even more preferably $C_1$-$C_6$ and most

preferably $C_1$-$C_4$-alkyl groups.

Alkenyl and alkynyl either alone or as part of a larger group (such as alkenylcarbonyl) moieties can be in the form of straight or branched chains with 2 to 6 carbon atoms, and the alkenyl moieties, where appropriate, can be of either the (E)- or (Z)-configuration. Examples are vinyl, allyl and propargyl. Alkenyl and alkynyl moieties can contain one or more double and/or triple bonds in any combination. It is understood, that allenyl and alkylinylalkenyl are included in these terms.

Halogen is fluorine, chlorine, bromine or iodine, preferably fluorine, chlorine or bromine.

Haloalkyl groups either alone or as part of a larger group (such as haloalkoxy, haloalkylthio) are alkyl groups which are substituted with one or more of the same or different halogen atoms and are, for example, $-CF_3$, $-CCl_3$, $-CFCl_2$, $-CF_2Cl$, $-CF_2Br$, $-CF_2H$, $FCH_2$-, $ClCH_2$-, $BrCH_2$-, $CH_3CHF$-, $CH_3CF_2$-, $CH_3CH_2CF_2$-, $CF_3CH_2$-, $CHF_2CH_2$-, $-CF_2CF_3$, $-CH(CH_3)CF_3$, $-CH(CF_3)_2$ or $-CF(CF_3)_2$, preferably $-CF_3$, $-CCl_3$, $-CFCl_2$, $-CF_2Cl$, $-CF_2Br$, $-CF_2H$, $FCH_2$-, $ClCH_2$-, $BrCH_2$-, $CH_3CHF$-, $CH_3CF_2$-, $CF_3CH_2$-, $CHF_2CH_2$-, $-CF_2CF_3$, $-CH(CH_3)CF_3$, $-CH(CF_3)_2$ or $-CF(CF_3)_2$.

Haloalkenyl are alkenyl groups which are substituted with one or more of the same or different halogen atoms and are, for example, $-CH=CF_2$, $-CH=CCl_2$, $-CF=CF_2$, $-CCl=CCl_2$, $-CH_2-CH=CF_2$, $-CH_2-CH=CCl_2$, $-CH_2-CF=CF_2$ or $-CH_2-CCl=CCl_2$.

In the context of the present specification the terms "aryl" either alone or as part of a larger group, (such as aryalkyl, aryloxy) "aromatic ring" and "aromatic ring system" refer to ring systems which may be mono-, bi- or tricyclic. Examples of such rings include phenyl, naphthalenyl, anthracenyl, indenyl or phenanthrenyl. A preferred aryl group is phenyl.

The terms "heteroaryl" and "hetaryl", either alone or as part of a larger group (such as heteroaryloxy), "heteroaromatic ring" or "heteroaromatic ring system" refer to an aromatic ring system containing at least one heteroatom and consisting either of a single ring or of two or more fused rings. Preferably, single rings will contain up to three and bicyclic systems up to four heteroatoms which will preferably be chosen from nitrogen, oxygen and sulphur. Examples of such groups include furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl. Preferred examples of heteroaromatic radicals include pyridyl, pyrimidyl, triazinyl, thienyl, furyl, oxazolyl, isoxazolyl, 2,1,3-benzoxadiazole and thiazolyl.

The terms heterocycle and heterocyclyl refer to a non-aromatic monocyclic or bicyclic ring system containing up to 10 atoms including one or more (preferably one or two) heteroatoms selected from O, S and N. Examples of such rings include 1,3-dioxolane, oxetane, tetrahydrofuran, morpholine, thiomorpholine and piperazine.

When present, the optional substituents on heterocyclyl include halogen, $C_1$-$C_6$-alkyl, $C_1$-$C_6$ - haloalkyl, $C_1$-$C_6$-alkoxy, $C_1$-$C_6$-haloalkoxy, $C_1$-$C_6$-alkoxyalkyl, $C_1$-$C_6$-alkoxycarboilyl, $C_1$-$C_6$-alkylcarbonyl, amino, cyano, $C_1$-$C_6$-alkylamino or di-$C_1$-$C_6$-alkylamino.

The term cycloalkyl includes $C_3$-$C_6$-cycloalkyl, namely cyclopropyl, cyclobutyl, cyclopentyl and cyclohexyl, preferably cyclopropyl, cyclopentyl, and cyclohexyl. When present, the optional substituents on cycloalkyl include halogen, $C_1$-$C_6$-alkyl, $C_1$-$C_6$-haloalkyl, $C_1$-$C_6$-alkoxy, $C_1$-$C_6$-haloalkoxy, $C_1$-$C_6$-alkoxyalkyl, $C_1$-$C_6$-alkoxycarbonyl, $C_1$-$C_6$-alkylearbonyl, amino, cyano, $C_1$-$C_6$-alkylamino or di-$C_1$-$C_6$-alkylamino.

When present, the optional substituents on aryl or heteroaryl are independently selected from halogen, nitro, cyano, rhodano, isothiocyanato, $C_1$-$C_6$-alkyl, $C_1$-$C_6$-haloalkyl, $C_1$-$C_6$-alkoxy-$C_1$-$C_6$-alkyl, $C_2$-$C_6$-alkenyl, $C_2$-$C_6$-haloalkenyl, $C_2$-$C_6$-alkynyl, $C_3$-$C_7$-cycloalkyl (itself optionally substituted with $C_1$-$C_6$-alkyl or halogen), $C_5$-$C_7$-cycloalkenyl (itself optionally substituted with $C_1$-$C_6$-alkyl or halogen), hydroxy, $C_1$-$C_{10}$-alkoxy, $C_1$-$C_{10}$-alkoxy-$C_1$-$C_{10}$-alkoxy, tri-$C_1$-$C_4$-alkyl-silyl-$C_1$-$C_6$-alkoxy, $C_{1-6}$ alkoxycarbonyl-$C_1$-$C_{10}$-alkoxy, $C_1$-$C_{10}$-haloalkoxy, aryl-$C_1$-$C_4$-alkoxy (where the aryl group is optionally substituted with halogen or $C_1$-$C_6$-alkyl), $C_3$-$C_7$-cycloalkyloxy (where the cycloalkyl group is optionally substituted with $C_1$-$C_6$-alkyl or halogen), $C_2$-$C_{10}$-alkenyloxy, $C_2$-$C_{10}$-alkynyloxy, SH, $C_1$-$C_{10}$-alkyltliio, $C_1$-$C_{10}$-halbalkylthio, aryl-$C_1$-$C_4$-alkylthio $C_3$-$C_7$-cycloalkylthio (where the cycloalkyl group is optionally substituted with $C_1$-$C_6$-alkyl or halogen), tri-$C_1$-$C_4$-alkylsilyl-$C_1$-$C_6$-alkylthio, arylthio, $C_1$-$C_6$-alkylsulfonyl, $C_1$-$C_6$-haloalkylsulfonyl, $C_1$-$C_6$-alkylsulfinyl, $C_1$-$C_6$-haloalkylsulfinyl, arylsulfonyl, tri-$C_1$-$C_4$-alkylsilyl, aryidi-$C_1$-$C_4$-alkylsilyl, $C_1$-$C_4$-alkyldiaryisilyl, triarylsilyl, $C_1$-$C_{10}$-alkylcarbonyl, $HO_2C$, $C_1$-$C_{10}$-alkoxycarbonyl, aminocarbonyl, $C_1$-$C_6$-alkylaminocarbonyl, di-$C_1$-$C_6$-alkyl-aminocarbonyl, N-$C_1$-$C_3$-alkyl-N-$C_1$-$C_3$-alkoxy)aminocarbonyl, $C_1$-$C_6$-alkylcarbonyloxy, arylcarbonyloxy, di-$C_1$-$C_6$-alkylamino-carbonyloxy, aryl (itself optionally substituted with $C_1$-$C_6$-alkyl or halogen), heteroaryl (itself optionally substituted with $C_1$-$C_6$-alkyl or halogen), heterocyclyl (itself optionally substituted with $C_1$-$C_6$-alkyl or halogen), aryloxy (where the aryl group is optionally substituted with $C_1$-$C_6$-alkyl or halogen), heteroaryloxy (where the heteroaryl group is optionally substituted with $C_1$-$C_6$-alkyl or halogen), heterocyclyloxy (where the heterocyclyl group is optionally substituted with $C_1$-$C_6$-alkyl or halogen), amino, $C_1$-$C_6$-alkylamino, di-$C_1$-$C_6$-alkylamino, $C_1$-$C_6$-alkylcarbonylamino, N-$C_1$-$C_6$-alkylcarbo-

nyl-N-$C_1$-$C_6$-alkylamino, arylcarbonyl, (where the aryl group is itself optionally substituted with halogen or $C_1$-$C_6$-alkyl) or two adjacent positions on an aryl or heteroaryl system may be cyclised to form a $_5$, 6 or 7 membered carbocyclic or heterocyclic ring, itself optionally substituted with halogen or $C_1$-$C_6$-alkyl. Further substituents for aryl or heteroaryl include aryl carbonyl amino (where the aryl group is substituted by $C_1$-$C_6$-alkyl or halogen), $C_1$-$C_6$-alkyloxycarbonylamino, $C_1$-$C_6$-alkyloxycarbonyl-N-$C_1$-$C_6$-alkylamino, aryloxycarbonylamino (where the aryl group is substituted by $C_1$-$C_6$-alkyl or halogen), aryloxycarbonyl-N-$C_1$-$C_6$-alkylamino, (where the aryl group is substituted by $C_1$-$C_6$-alkyl or halogen), arylsulfonylamino (where the aryl group is substituted by $C_1$-$C_6$-alkyl or halogen), atylsulfonyl-N-$C_1$-$C_6$-alkylamino (where the aryl group is substituted by $C_1$-$C_6$-alkyl or halogen), aryl-N-$C_1$-$C_6$-alkylamino (where the aryl group is substituted by $C_1$-$C_6$-alkyl or halogen), arylamino (where the aryl group is substituted by $C_1$-$C_6$-alkyl or halogen), heteroaryl amino (where the heteroaryl group is substituted by $C_1$-$C_6$-alkyl or halogen), heterocyclylamino (where the heterocyclyl group is substituted by $C_1$-$C_6$ alkyl or halogen), aminocarbonyl amino, $C_1$-$C_6$-alkylaminocarbonyl amino, di-$C_1$-$C_6$-alkylaminocarbonyl amino, arylaminocarbonyl amino (where the aryl group is substituted by $C_1$-$C_6$-alkyl or halogen), aryl-N-$C_1$-$C_6$-alkylaminocarbonylamino (where the aryl group is substituted by $C_1$-$C_6$-alkyl or halogen), $C_1$-$C_6$-alkylaminocarbonyl-N-$C_1$-$C_6$-alkyl amino, di-$C_1$-$C_6$-alkylaminocarbonyl-N-$C_1$-$C_6$-alkyl amino, arylaminocarbonyl-N-$C_1$-$C_6$-alkyl amino (where the aryl group is substituted by $C_1$-$C_6$-alkyl or halogen) and aryl-N-$C_1$-$C_6$-alkylaminocarbonyl-N-$C_1$-$C_6$-alkyl amino (where the aryl group is substituted by $C_1$-$C_6$-alkyl or halogen).

For substituted phenyl moieties, heterocyclyl and heteroaryl groups, it is preferred that one or more substituents are independently selected from halogen, $C_1$-$C_6$-alkyl, $C_1$-$C_6$-haloalkyl, $C_1$-$C_6$-alkoxy-$C_1$-$C_6$-alkyl, $C_1$-$C_6$-alkoxy, $C_1$-$C_6$-haloalkoxy, $C_1$-$C_6$-alkylthio, $C_1$-$C_6$-haloalkylthio, $C_1$-$C_6$-alkylsulfinyl, $C_1$-$C_6$-haloalkylsulfinyl, $C_1$-$C_6$-alkylsulfonyl, $C_1$-$C_6$-haloalkylsulfonyl, $C_2$-$C_6$-alkenyl, $C_2$-$C_6$-haloalkenyl, $C_2$-$C_6$-alkynyl, $C_3$-$C_6$-cycloalkyl, nitro, cyano, $CO_2H$, $C_1$-$C_6$-alkylcarbonyl, $C_1$-$C_6$-alkoxycarbonyl, aryl, heteroaryl, $R^{50}R^{51}N$ or $R^{52}R^{53}C(O)$; wherein $R^{50}$, $R^{51}$, $R^{52}$ and $R^{53}$ are, independently, hydrogen or $C_1$-$C_6$-alkyl.

It is to be understood that dialkylamino substituents include those where the dialkyl groups together with the N atom to which they are attached form a five, six or seven-membered heterocyclic ring which may contain one or two further heteroatoms selected from O, N or S and which is optionally substituted by one or two independently selected $C_1$-$C_6$-alkyl groups. When heterocyclic rings are formed by joining two groups on an N atom, the resulting rings are suitably pyrrolidine, piperidine, thiomorpholine and morpholine each of which may be substituted by one or two independently selected $C_1$-$C_{-6}$alkyl groups. Preferably the optional substituents on an alkyl moiety include one or more of halogen, nitro, cyano, $HO_2C$, $C_1$-$C_{10}$-alkoxy (itself optionally substituted by $C_{1}$-$_{10}$-alkoxy), aryl-$C_1$-$C_4$-alkoxy, $C_1$-$C_{10}$-alkylthio, $C_1$-$C_{10}$-alkylcarbonyl, $C_3$-$C_5$-cycloalkylcarbonyl, $C_1$-$C_{10}$-alkoxycarbonyl, $C_1$-$C_6$-alkylaminocarbonyl, di-$C_1$-$C_6$-alkylaminocarbonyl, $C_1$-$C_6$-alkylcarbonyloxy, optionally substituted phenyl, heteroaryl, aryloxy, arylcarbonyloxy, heteroaryloxy, heterocyclyl, heterocyclyloxy, $C_3$-$C_7$-cycloalkyl (itself optionally substituted with $C_1$-$C_6$-alkyl or halogen), $C_3$-$C_7$-cycloalkyloxy, $C_5$-$C_7$-cycloalkenyl, $C_1$-$C_6$-alkylsulfonyl, $C_1$-$C_6$-alkylsulfinyl, tri-$C_1$-$C_6$-alkylsilyl, tri-$C_1$-4-alkylsilyl-$C_1$-$C_6$-alkoxy, aryldi-$C_1$-$C_6$-alkylsilyl, $C_1$-$C_4$-alkyldiarylsilyl and triarylsilyl.

Preferably the optional substituents on alkenyl include one or more of halogen, $C_1$-$C_{10}$-alkyl, $C_1$-$C_{10}$-alkoxy, $C_1$-$C_{10}$-haloalkoxy, aryl and $C_3$-$C_7$-cycloalkyl.

Preferably the optional substituents on alkynyl include one or more of $C_1$-$C_{10}$-alkyl, $C_1$-$C_{10}$-alkoxy, $C_1$-$C_{10}$-haloalkoxy, aryl and $C_3$-$C_7$-cycloalkyl.

A preferred optional substituent for heterocyclyl is $C_1$-$C_3$-alkyl.

Preferably the optional substituents for cycloalkyl include halogen, cyano and $C_1$-$C_6$-alkyl.

The formula (I) provides a general definition of the compounds according to the invention.

Preferred substituents or ranges of the radicals given in the formulae mentioned above and below are illustrated below.

A is preferably selected from -$CHR^5$-, -$CHR^5CHR^6$-, -O-, -$S(O)_n$-, -$NR^7$-, -$OCHR^5$-, - $CHR^5O$-, -$S(O)_nCHR^5$-, -$CHR^5S(O)_n$-, -C(X)- and -C(=$NOR^7$)-;

B is preferably selected from -$CHR^8$-, -O- and -$S(O)_n$-;

X is preferably selected from oxygen and sulphur;

n is an integer preferably selected from 0, 1 and 2;

R, $R^1$, $R^2$ and $R^3$ are independently preferably selected from hydrogen, halogen, hydroxy, $C_1$-$C_6$-alkyl, $C_1$-$C_6$-alkoxy, $C_1$-$C_6$-alkylthio, $C_1$-$C_6$-haloalkyl, $C_1$-$C_6$-haloalkoxy, $C_1$-$C_6$-haloalkylthio, $C_2$-$C_6$-alkenyl, $C_2$-$C_6$-haloalkenyl, $C_2$-$C_6$-alkynyl, cyano, nitro, phenyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), phenyloxy (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_1$-$C_6$-alkoxy-$C_1$-$C_6$-alkyl, $C_1$-$C_6$-alkoxy-$C_1$-$C_6$-alkoxy-$C_1$-$C_6$-alkyl,

$C_3$-$C_6$-cycloalkyl, cyano-$C_1$-$C_6$-alkyl, formyl, $C_1$-$C_6$-alkoxycarbonyl, $C_1$-$C_6$-alkylcarbonyl, $C_2$-$C_6$-alkenylcarbonyl, di-$C_1$-$C_6$-alkylphosphonato, amino, mono- and di-$C_1$-$C_6$-alkylamino, $C_3$-$C_6$-cycloalkylamino, ($C_3$-$C_6$-cycloalkyl)($C_1$-$C_6$-alkyl)amino, $C_1$-$C_6$-alkylsulfonyl, $C_1$-$C_6$-haloalkylsulfonyl, $C_1$-$C_6$-alkylsulfinyl, $C_1$-$C_6$-haloalkylsulfinyl, $C_1$-$C_6$-alkylsulfinyl-$C_1$-$C_6$-alkyl, $C_1$-$C_6$-alkylsulfonyl-$C_1$-$C_6$-alkyl, $C_1$-$C_6$-alkylthiocarbonyl and hydroxycarbonyl;

$R^4$ is preferably selected from hydrogen, amino, hydroxyl, cyano, $C_1$-$C_6$-alkyl, $C_1$-$C_6$-haloalkyl, $C_3$-$C_6$-cycloalkyl, phenyl-$C_1$-$C_6$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_2$-$C_6$-alkenyl, $C_2$-$C_6$-alkynyl, $C_3$-$C_6$-cycloalkyl-Cl-$C_6$-alkyl, $C_1$-$C_6$-alkoxy, -C(X)$R^{15}$, -C(X)O$R^{16}$, -C(X)S$R^{16}$, - $S(O)_nR^{16}$, -C(X)N$R^{17}R^{18}$, -$S(O)_n$N$R^{17}R^{18}$, -P(X)(O$R^{19}$)(O$R^{20}$), -P(X)(N$R^{21}R^{22}$)(N$R^{23}R^{24}$), -CH$R^{25}$-X-$R^{26}$, -CH=N$R^{27}$ and -C(=N$R^{31}$)(S$R^{32}$);

$R^5$ is preferably selected from hydrogen, halogen, hydroxy, $C_1$-$C_6$-alkyl, $C_1$-$C_6$-alkoxy, $C_1$-$C_6$-alkylthio, $C_1$-$C_6$-haloalkyl, $C_1$-$C_6$-haloalkoxy, cyano and nitro;

$R^6$ is preferably selected from hydrogen, halogen, hydroxy, $C_1$-$C_6$-alkyl, $C_1$-$C_6$-alkoxy, $C_1$-$C_6$-alkylthio, $C_1$-$C_6$-haloalkyl, $C_1$-$C_6$-haloalkoxy, cyano and nitro;

$R^7$ is preferably selected from hydrogen and $C_1$-$C_6$-alkyl;

$R^8$ is preferably selected from hydrogen, halogen, $C_1$-$C_6$-alkyl and $C_1$-$C_6$-alkoxy;

$R^9$ is preferably selected from hydrogen, amino, hydroxyl, $C_1$-$C_6$-alkyl, $C_1$-$C_6$-haloalkyl, $C_1$-$C_6$-alkoxy-$C_1$-$C_6$-alkyl, hydroxy-$C_1$-$C_6$-alkyl, $C_1$-$C_6$-alkoxy, $C_3$-$C_6$-cycloalkyl, phenyl-$C_1$-$C_6$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_2$-$C_6$-alkenyl, $C_2$-$C_6$-alkynyl, $C_3$-$C_6$-cycloalkyl-$C_1$-$C_6$-alkyl, $C_1$-$C_6$-alkylcarbonyl, $C_1$-$C_6$-alkoxycarbonyl, $C_1$-$C_6$-alkylamino(thio)carbonyl and di-$C_1$-$C_6$-alkylamino (thio)carbonyl;

$R^{10}$, $R^{11}$, $R^{12}$ and $R^{13}$ are independently preferably selected from hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, $C_3$-$C_6$-cycloalkyl, $C_3$-$C_6$-cycloalkyl-$C_1$-$C_6$-alkyl, $C_2$-$C_6$-alkenyl, phenyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), phenyl-$C_1$-$C_6$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), heteroaryl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), heteroaryl-$C_1$-$C_6$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy);

$R^{14}$ is preferably selected from hydrogen, $C_1$-$C_6$-alkyl, $C_1$-$C_6$-haloalkyl, $C_1$-$C_6$-alkoxy-$C_1$-$C_6$-alkyl, hydroxy-$C_1$-$C_6$-alkyl, $C_3$-$C_6$-cycloalkyl, phenyl-$C_1$-$C_6$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_2$-$C_6$-alkenyl, $C_2$-$C_6$-alkynyl, $C_3$-$C_6$-cycloalkyl-$C_1$-$C_6$-alkyl, $C_1$-$C_6$-alkylcarbonyl, $C_1$-$C_6$-alkoxycarbonyl, $C_1$-$C_6$-alkylamino(thio)carbonyl, di-$C_1$-$C_6$-alkylamino(thio)carbonyl, phenylcarbonyl (optionally substituted by hydrogen, halogen, cyano, nitro, $C_1$-$C_3$ alkyl, $C_1$-$C_3$ haloalkyl, $C_1$-$C_3$ alkoxy, $C_1$-$C_3$ haloalkoxy), phenyloxycarbonyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy);

$R^{15}$ is preferably selected from hydrogen and $C_1$-$C_6$-alkyl;

$R^{16}$ is preferably selected from hydrogen, $C_1$-$C_6$-alkyl, $C_1$-$C_6$-haloalkyl, $C_1$-$C_6$-hydroxyalkyl, hydroxycarbonyl-$C_1$-$C_6$-alkyl and $C_1$-$C_6$-alkoxycarbonyl-$C_1$-$C_6$-alkyl;

$R^{17}$ and $R^{18}$ are independently preferably selected from hydrogen, $C_1$-$C_6$-alkyl, $C_1$-$C_6$-haloalkyl, $C_3$-$C_6$-cycloalkyl, $C_1$-$C_6$-cyanoalkyl, phenyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_1$-$C_6$-alkylcarbonyl, $C_1$-$C_6$-alkoxycarbonyl, $C_1$-$C_6$-alkoxycarbonyl-$C_1$-$C_6$-alkyl, $C_1$-$C_6$-alkoxy-$C_1$-$C_6$-alkyl, $C_1$-$C_6$-alkylthio-$C_1$-$C_6$-alkyl, $C_1$-$C_6$-alkylsulfinyl-$C_1$-$C_6$-alkyl, $C_1$-$C_6$-alkylsulfonyl-$C_1$-$C_6$-alkyl, mono- or di-$C_1$-$C_6$-alkylaminocarbonyl-$C_1$-$C_6$-alkyl, $C_1$-$C_6$-aminoalkyl, mono- or di-$C_1$-$C_6$-alkylamino-$C_1$-$C_6$-alkyl and phenylcarbonyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy);

$R^{19}$ and $R^{20}$ are independently preferably selected from hydrogen, $C_1$-$C_6$-alkyl and $C_1$-$C_6$-haloalkyl;

$R^{21}$, $R^{22}$, $R^{23}$ and $R^{24}$ are independently preferably selected from hydrogen and $C_1$-$C_6$-alkyl;

$R^{25}$ is preferably selected from hydrogen and $C_1$-$C_6$-alkyl;

$R^{26}$ is preferably selected from $C_1$-$C_6$-alkyl, $C_1$-$C_6$-haloalkyl, -C(X)$R^{28}$ and -P(X)($OR^{29}$)($OR^{30}$);

$R^{27}$ is preferably selected from hydrogen, $C_1$-$C_6$-alkyl, $C_1$-$C_6$-haloalkyl, hydroxy, $C_1$-$C_6$-alkoxy, $C_1$-$C_6$-haloalkoxy or cyano;

$R^{28}$ is preferably selected from hydrogen, $C_1$-$C_6$-alkyl and $C_1$-$C_6$-haloalkyl; ,

$R^{29}$ and $R^{30}$ are independently preferably selected from hydrogen, $C_1$-$C_6$-alkyl and $C_1$-$C_6$-haloalkyl;

$R^{31}$ and $R^{32}$ are independently preferably selected from hydrogen, $C_1$-$C_6$-alkyl, phenyl-$C_1$-$C_6$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), -C(X)$R^{15}$, -$CO_2R^{16}$, -$S(O)_nR^{16}$, -C(X)$NR^{17}R^{18}$, -$S(O)_nNR^{17}R^{18}$, -P(X)($OR^{19}$)($OR^{20}$), - P(X)($NR^{21}R^{22}$)($NR^{23}R^{24}$) and -$CHR^{25}$-X-$R^{26}$;

A is more preferably selected from -$CHR^5$-, -$CHR^5CHR^6$-, -O-, -$S(O)_n$-, -$NR^7$-, -$OCHR^5$-, - $CHR^5O$-, -$S(O)_nCHR^5$-, -$CHR^5S(O)_n$-, -C(X)- or -C(=$NOR^7$)-;
B is more preferably selected from -$CHR^8$-, -O- or -$S(O)_n$-;
X is more preferably selected from oxygen or sulphur;
n is an integer more preferably selected from 0, 1 or 2;

R, $R^1$, $R^2$ and $R^3$ are independently more preferably selected from hydrogen, halogen, hydroxy, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, $C_1$-$C_4$-haloalkylthio, nitro, cyano, $C_1$-$C_4$-alkoxy-$C_1$-$C_4$-alkyl, cyano-$C_1$-$C_4$-alkyl, formyl, $C_1$-$C_4$-alkoxycarbonyl, $C_1$-$C_4$-alkylcarbonyl, amino, mono- and di-$C_1$-$C_4$-alkylamino, $C_3$-$C_6$-cycloalkylamino, ($C_3$-$C_6$-cycloalkyl)($C_1$-$C_4$-alkyl)amino, $C_1$-$C_4$-alkylsulfonyl, $C_1$-$C_4$-haloalkylsulfonyl,$C_1$-$C_4$-alkylsulfinyl,$C_1$-$C_4$-haloalkylsulfinyl$C_1$-$C_4$-alkylsulfinyl-$C_1$-$C_4$-alkyl,$C_1$-$C_4$-alkylsulfonyl-$C_1$-$C_4$-alkyl, $C_1$-$C_4$-alkylthiocarbonyl and hydroxycarbonyl;

$R^4$ is more preferably selected from hydrogen, amino, hydroxyl, $C_1$-$C_4$-alkyl, $C_1$-$C_4$-haloalkyl, $C_3$-$C_6$-cycloalkyl, phenyl-$C_1$-$C_4$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_2$-$C_4$-alkenyl, $C_2$-$C_4$-alkynyl, $C_3$-$C_6$-cycloalkyl, $C_1$-$C_4$-alkoxy, -C(X)$R^{15}$, -C(X)$OR^{16}$, -$S(O)_nR^{16}$ and -$CHR^{25}$-X-$R^{26}$;

$R^5$ is more preferably selected from hydrogen, halogen, hydroxy, $C_1$-$C_4$-alkyl and $C_1$-$C_4$-alkoxy;

$R^6$ is more preferably selected from hydrogen, halogen, hydroxy, $C_1$-$C_4$-alkyl and $C_1$-$C_4$-alkoxy;

$R^7$ is more preferably selected from hydrogen and $C_1$-$C_4$-alkyl;

$R^8$ is more preferably selected from hydrogen, halogen, $C_1$-$C_4$-alkyl and $C_1$-$C_4$-alkoxy;

$R^9$ is more preferably selected from hydrogen, amino, hydroxyl, $C_1$-$C_4$-alkyl, $C_1$-$C_4$-haloalkyl, $C_1$-$C_4$-alkoxyalkyl, hydroxy-$C_1$-$C_4$-alkyl, $C_1$-$C_4$-alkoxy, $C_3$-$C_6$-cycloalkyl, phenyl-$C_1$-$C_4$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_2$-$C_4$-alkenyl, $C_2$-$C_4$-alkynyl, $C_3$-$C_6$-cycloalkyl-$C_1$-$C_4$-alkyl, $C_1$-$C_4$-alkylcarbonyl, $C_1$-$C_4$-alkoxycarbonyl, $C_1$-$C_4$-alkylamino(thio)carbonyl and di-$C_1$-$C_4$-alkylamino (thio)carbonyl;

$R^{10}$, $R^{11}$, $R^{12}$ and $R^{13}$ are independently more preferably selected from hydrogen and $C_1$-$C_4$-alkyl;

$R^{14}$ is more preferably selected from hydrogen, $C_1$-$C_4$-alkyl, $C_1$-$C_4$-haloalkyl, $C_1$-$C_4$-alkoxy-$C_1$-$C_4$-alkyl, hydroxy-$C_1$-$C_4$-alkyl, $C_3$-$C_6$-cycloalkyl, phenyl-$C_1$-$C_4$-alkyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy), $C_2$-$C_4$-alkenyl, $C_2$-$C_4$-alkynyl, $C_1$-$C_4$-alkylcarbonyl, $C_1$-$C_4$-alkoxycarbonyl, $C_1$-$C_4$-alkylamino(thio)carbonyl, di-$C_1$-$C_4$-alkylamino(thio)carbonyl, phenylcarbonyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy) and phenyloxycarbonyl (optionally substituted by halogen, cyano, nitro, $C_1$-$C_3$ alkyl, $C_1$-$C_3$ haloalkyl, $C_1$-$C_3$ alkoxy, $C_1$-$C_3$ haloalkoxy);

$R^{15}$ is more preferably selected from hydrogen and $C_1$-$C_4$-alkyl;

$R^{16}$ is more preferably selected from hydrogen and $C_1$-$C_4$-alkyl,;

$R^{25}$ is more preferably selected from hydrogen and $C_1$-$C_4$-alkyl;

$R^{26}$ is more preferably selected from $C_1$-$C_4$-alkyl, $C_1$-$C_4$-haloalkyl and -C(X)$R^{28}$;

$R^{28}$ is more preferably selected from hydrogen, $C_1$-$C_4$-alkyl and $C_1$-$C_4$-haloalkyl.

A preferred group of compounds of formula (I) are those in which A is -$CHR^5$-, B is -$CHR^8$- and $R^4$ and $R^{11}$ to $R^{13}$ are hydrogen.

Another preferred group of compounds of formula (I) are those in which A is -O -, B is -$CHR^8$- and $R^4$ and $R^{11}$ to $R^{13}$ are hydrogen.

Another preferred group of compounds of formula (I) are those in which A is -$OCHR^5$-, B is - $CHR^8$- and $R^4$ and $R^{11}$ to $R^{13}$ are hydrogen.

Another preferred group of compounds of formula (I) are those in which A is -$CH_2CH_2$-, B is - $CH_2$-, $R^4$ and $R^{11}$ to $R^{13}$ are hydrogen.

Another preferred group of compounds of formula (I) are those in which A is $SCH_2$-, B is -$CH_2$- and $R^4$ and $R^{11}$ to $R^{13}$ are hydrogen.

In the context of the present invention, the (S)-Enantiomers of the compounds of formula (I) represent a preferred group of compounds, if

A is selected from -$CHR^5$-, -$CHR^5CHR^6$-, -$OCHR^5$-, -$S(O)_nCHR^5$-, -C(X)- or -C(=$NOR^7$)-;
B is selected from -$CHR^8$-.

In the context of the present invention, the (R)-Enantiomers of the compounds of formula (I) represent a preferred group of compounds, if

A is selected from -$CHR^5$-, -$CHR^5CHR^6$-, -O-, -$S(O)_n$-, -$NR^7$-, -$OCHR^5$-, -$CHR^5O$-, - $S(O)_nCHR^5$-, -$CHR^5S(O)_n$-, -C (X)- or -C(=$NOR^7$)-;
B is selected from -O- or -$S(O)_n$-.

In the context of the present invention, the (R)-Enantiomers of the compounds of formula (I) represent a preferred group of compounds, if

A is selected from -O-, -$S(O)_n$-, -$NR^7$-, -$CHR^5O$-, -$CHR^5S(O)_n$-;
B is selected from -$CHR^8$-.

The relative stereochemistry is determined according to the so called Cahn-Ingold-Prelog priority rules and leads to (S) or (R) enantiomers.

If, in Process 1 according to the invention for preparing the compounds of the formula (I-a), the compound of the formula (II) or (II-a) is, for example, (1S)-4-fluoroindanyl-1-isothiocyanate, and the compound of formula (III) is 2-aminoethanol, the preparation process 1 can be represented by the reaction scheme as shown below:

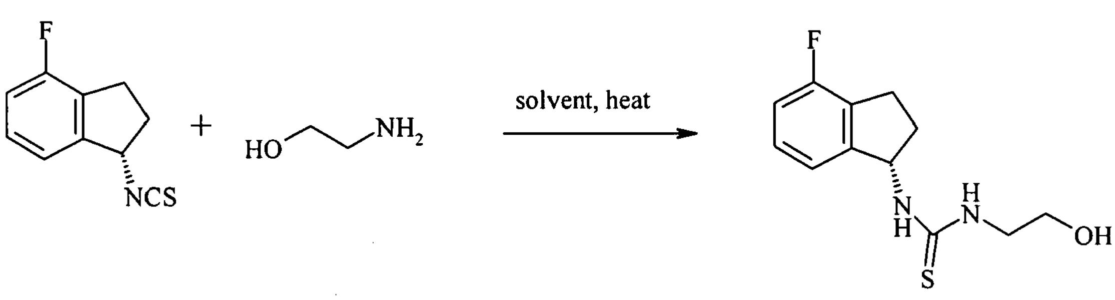


Formulae (II) and (11-a) provide a general definition of the compounds required as starting materials for carrying

out the process 1 according to the invention.

In formulae (II) and (II-a), R, R1 to R3, A and B represent those radicals which have already been mentioned as substituents in connection with the description of the compounds of the formula (I).

The compounds of formula (II) are known and/or can be obtained by methods known from the literature, e.g. W02007/020377.

Furthermore, the present invention provides novel chiral intermediates of formula (II-a):

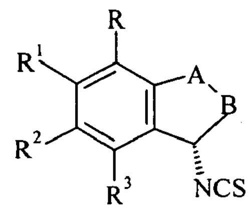

(II-a)

in which

A, B, R, $R^1$, $R^2$ and $R^3$ are as defined as above, except the compound in which R = $R^1$ = $R^2$ = $R^3$ = hydrogen and A = $CH_2$ and B = $CH_2$, which compound is already known from US200604019.

A preferred group of compounds of formula (II-a) are those in which A is -O-and B is -$CHR^8$-.

Another preferred group of compounds of formula (II-a) are those in which A is -$OCHR^5$- and B is -$CHR^8$-.

Another preferred group of compounds of formula (II-a) are those in which A is -$CHR^5CHR^6$- and B is -$CHR^8$-.

Another preferred group of compounds of formula (II-a) are those in which A is -$S(O)_nCHR^5$- and B is -$CHR^8$-.

The relative stereochemistry is depicted according to the so called Cahn-Ingold-Prelog priority rules and leads to (S) or (R) enantiomers.

It has been found, that isothiocyanates (II-a) can be obtained by the general processes shown in Scheme 4. The chiral amines are available according to literature, e.g. J. Org. Chem., 2006, 71(18),

Formula (III) provides a general definition of the compounds required as starting materials for carrying out Process 1 according to the invention.

In formula (III), $R^9$ to $R^{14}$ represent those radicals which have already been mentioned as substituents in connection with the description of the compounds of the formula (I).

The compounds of formula (III) are known and/or can be obtained by methods known from the literature or are commercial available (Synthetic Communications (1992), 22(18), 2703-2710; Journal of Chemical Research, Synopses (1991), (8), 201; US 2425615; Chemische Berichte (1964), 97(2), 510-519).

When carrying out Process 1 according to the invention, the reaction component of the formula (II) and the compound of formula (III) are generally employed in about equimolar amounts. However, it is also possible to use a relatively large excess (up to 3 equivalents) of one component or the other.

If, in Process 2 according to the invention for preparing the compounds of the formula (I-b), the compound of the formulae (IV) or (IV-a) is, for example, (1S)-4-fluoroindanyl-1-amine, and the compound of formula (V) is 1-methoxy-2-ethyl amine, the preparation process 2 can be represented by the following reaction scheme:

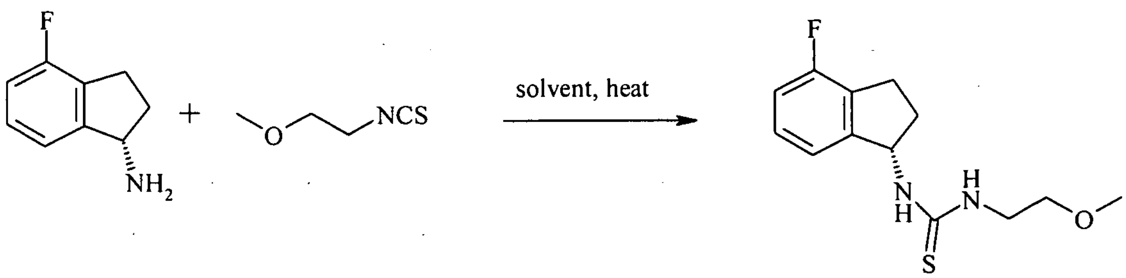

Formulae (IV) and (IV-a) provide a general definition of the compounds required as starting materials for carrying out the process 2 according to the invention.

In formulae (IV) and (IV-a), R, $R^1$ to $R^3$, $R^4$, A and B represent those radicals which have already been mentioned as substituents in connection with the description of the compounds of the formula (I).

The compounds of formula (IV) are known and/or can be obtained by methods known from the literature, e.g. W02007/020377, EP538314, US5538988.

The chiral amines (IV-a) are available according to literature, e.g. J. Org. Chem., 2006, 71(18), 6859 or Chem. Eur. J., 2000, 6(10), 1840.

Formula (V) provides a general definition of the compounds required as starting materials for carrying out Process 2 according to the invention.

In formula (V), $R^{10}$ to $R^{14}$ represent those radicals which have already been mentioned as substituents in connection with the description of the compounds of the formula (I).

The compounds of formula (V) are known and/or can be obtained by methods known from the literature or are commercial available (JP 37015645; Journal of Carbohydrate Chemistry (1995), 14(8), 1133-1152; Czech. Collection of Czechoslovak Chemical Communications (1986), 51(1), 112-117).

When carrying out Process 2 according to the invention, the compounds of formulae (IV) or (IV-a) and the compound of formula (V) are generally employed in about equimolar amounts. However, it is also possible to use a relatively large excess (up to 3 equivalents) of one component or the other.

If, in Process 3 according to the invention for preparing the compounds of the formula (I), the compound of the formulae (IV) or (IV-a) is, for example, (1S)-4-fluoroindanyl-1-amine, and the compound of formula (III) is 1-aminoethanol, the preparation process 3 can be represented by the reaction scheme as shown below:

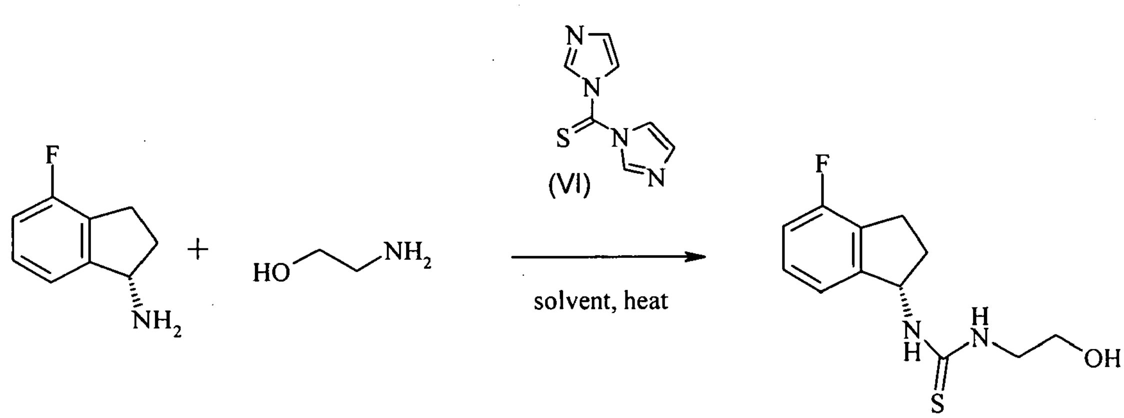


Formulae (IV), (IV-a) and (III) provide a general definition of the compounds required as starting materials for carrying out the process 2 according to the invention.

In formulae (IV) and (IV-a), R, $R^1$ to $R^3$, $R^4$, A and B represent those radicals which have already been mentioned as substituents in connection with the description of the compounds of the formula (I).

The compounds of formula (IV) are known and/or can be obtained by methods known from the literature, e.g. W02007/020377, EP538314, US5538988.

The chiral amines (IV-a) are available according to literature, e.g. J. Org. Chem., 2006, 71(18), 6859 or Chem. Eur. J., 2000, 6(10), 1840.

The formula (III) provides a general definition of the compounds required as starting materials for carrying out Process 3 according to the invention.

In formula (III), $R^9$ to $R^{14}$ represent those radicals which have already been described as substituents in connection with the description of the compounds of the formula (I).

The compounds of formula (III) are known and/or can be obtained by methods known from the literature or are commercial available.

Thiocarbonyl diimidazole (VI) as thiocarbonylating agent is commercial available.

When carrying out Process 3 according to the invention, the reaction component of the formulae (IV) or (IV-a) and the compounds of formula (III) are generally employed in about equimolar amounts. However, it is also possible to use a relatively large excess (up to 3 equivalents) of one component or the other.

If, in Process 4 according to the invention for preparing the intermediates of the formula (II-a), the compounds

of the formula (IV-a) is, for example, (1S)-4-fluoroindanyl-1-amine, the preparation process 4 can be represented by the reaction schemes as shown below:

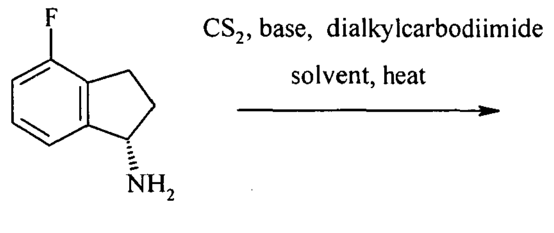


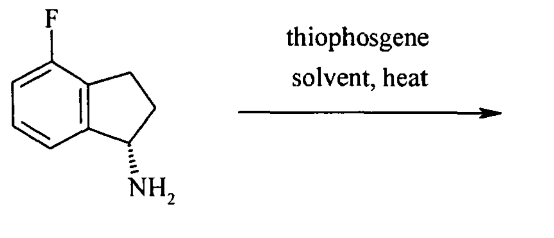


Formula (IV-a ) provides a general definition of the compounds required as starting materials for carrying out the process 4 according to the invention.

In formula (IV-a), R, $R^1$ to $R^3$, $R^4$, A and B represent those radicals which have already been described as substituents in connection with the description of the compounds of the formula (I).

The chiral amines (IV-a) are available according to literature, e.g. J. Org. Chem., 2006, 71(18), 6859 or Chem. Eur. J., 2000, 6(10), 1840.

Synthesis of Isothiocyanates is generally known and described in e.g. DE 2505301 and US 4,005,140.

Suitable bases for the first process shown above include e.g. triethylamine.

Suitable dialkylcarbodiimides include dicyclohexylcarbodiimide (DCC).

In general, it is advantageous to carry out Processes 1- 4 according to the invention in the presence of diluents.

Diluents are advantageously employed in such an amount that the reaction mixture remains readily stirable during the entire process. Suitable diluents for carrying out Process 1 - 4 according to the invention are all organic solvents which are inert under the reaction conditions.

Examples which may be mentioned are: halogenated hydrocarbons, in particular chlorinated hydrocarbons, such as tetrachloroethylene, tetrachloroethane, dichloropropane, methylene chloride, dichlorobutane, chloroform, carbon tetrachloride, trichloroethane, trichloroethylene, penta-chloroethane, difluorobenzene, 1,2-dichloroethane, chlorobenzene, bromobenzene, dichlorobenzene, chlorotoluene, trichlorobenzene; alcohols, such as methanol, ethanol, isopropanol, butanol; ethers, such as ethyl propyl ether, methyl tert-butyl ether, n-butyl ether, anisole, phenetole, cyclohexyl methyl ether, dimethyl ether, diethyl ether, dipropyl ether, diisopropyl ether, di-n-butyl ether, diisobutyl ether, diisoamyl ether, ethylene glycol dimethyl ether, tetrahydrofuran, dioxane, dichlorodiethyl ether and polyethers of ethylene oxide and/or propylene oxide; nitrated hydrocarbons, such as nitromethane, nitroethane, nitropropane, nitrobenzene, chloronitrobenzene, o-nitrotoluene; nitriles, such as acetonitrile, propionitrile, butyronitrile, isobutyronitrile, benzonitrile, m-chlorobenzonitrile and also compounds such as tetrahydrothiophene dioxide and dimethyl sulfoxide, tetramethylene sulfoxide, dipropyl sulfoxide, benzyl methyl sulfoxide, diisobutyl sulfoxide, dibutyl sulfoxide, diisoamyl sulfoxide; sulfones, such as dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone and pentamethylene sulfone; aliphatic, cycloaliphatic or aromatic hydrocarbons, such as pentane, hexane, heptane, octane, nonane and industrial hydrocarbons, for example white spirits with components having boiling points in the range of, for example, from 40°C to 250°C, cymene, petroleum fractions having a boiling point interval of from 70°C to 190°C, cyclohexane, methylcyclohexane, petroleum ether, ligroin, octane, benzene, toluene, chlorobenzene, bromobenzene, nitrobenzene, xylene; esters, such as methyl acetate, ethyl acetate, butyl acetate, isobutyl acetate, and also dimethyl carbonate, dibutyl carbonate, ethylene carbonate; amides, such as hexamethylenephosphoric triamide, formamide, *N*-methylformamide, *N,N*-dimethylformamide, *N,N*-dipropylformamide, *N,N*-dibutylformamide, *N*-methylpyrrolidine, *N*-methylcaprolactam, 1,3-dimethyl-3,4,5,6-tetrahydro-2(1H)-pyrimidine, octylpyrrolidone, octylcaprolactam, 1,3-dimethyl-2-imidazolinedione, *N*-formylpiperidine, *N,N*'-1,4-diformylpiperazine; ketones, such as acetone, acetophenone, methyl ethyl ketone, methyl butyl ketone.

It is, of course, also possible to use mixtures of the solvents and diluents mentioned for the process according to the invention.

However, preferred diluents for carrying out the preparation process according to the invention are

- methylene chloride, chloroform, tetrachloroethane;
- methyl tert-butyl ether, diisopropyl ether, tetrahydrofurane, dioxane;
- acetonitrile, propionitrile, butyronitrile;
- dimethyl sulfoxide;
- pentane, hexane, heptane, octane, industrial hydrocarbons, for example white spirits with components having boiling points in the range of, for example, from 40˚C to 250˚C, cymene, petroleum fractions having a boiling point interval of from 70˚C to 190˚C, cyclohexane ligroin, petroleum ether, benzene, toluene, chlorobenzene, xylenes;
- methyl acetate, ethyl acetate;
- *N,N* dimethylformamide, *N*-methylpyrrolidine;
- acetone.

When carrying the Process 1 - 4 according to the invention, the reaction temperature can be varied within a relatively wide range. In general, the process is carried out at temperatures between 0˚C and 250˚C, preferably between 50˚C and 150˚C. Process 1 according to the invention is generally carried out under atmospheric pressure.

After the reaction has gone to completion, the entire reaction mixture is concentrated. The products obtained after work-up can be purified in a customary manner by recrystallization, distillation under reduced pressure or column chromatography (cf. also the Preparation Examples).

Racemates or diastereomer mixtures can be separated by column chromatography, if appropriate on a suitable chiral phase, into the corresponding enantiomers or individual diastereomers. The absolute configuration can be determined with the aid of X-ray structure analysis after crystallization of the substance (cf. also the Preparation Examples).

The active insecticidal compounds of this invention may be formulated and/or applied with one or more additional compound. Such combinations may provide certain advantages, such as, without limitation, exhibiting synergistic effects for greater control of insect pests, reducing rates of application of insecticide thereby minimizing any impact to the environment and to worker safety, controlling a broader spectrum of insect pests, safening of crop plants to phytotoxicity, and improving tolerance by non-pest species, such as mammals and fish.

Additional compounds include, without limitation, other pesticides, plant growth regulators, fertilizers, soil conditioners, or other agricultural chemicals. In applying an active compound of this invention, whether formulated alone or with other agricultural chemicals, an effective amount and concentration of the active compound is of course employed; the amount may vary in the range of, e.g. about 0.001 to about 3 kg/ha, preferably about 0.03 to about 1 kg/ha. For field use, where there are losses of insecticide, higher application rates (e.g., four times the rates mentioned above) may be employed.

The active compounds according to the invention, in combination with good plant tolerance and favourable toxicity to warm-blooded animals and being tolerated well by the environment, are suitable for protecting plants and plant organs, for increasing the harvest yields, for improving the quality of the harvested material and for controlling animal pests, in particular insects, arachnids, helminths, nematodes and molluscs, which are encountered in agriculture, in horticulture, in animal husbandry, in forests, in gardens and leisure facilities, in the protection of stored products and of materials, and in the hygiene sector. They may be preferably employed as plant protection agents. They are active against normally sensitive and resistant species and against all or some stages of development. The abovementioned pests include:

From the order of the Anoplura (Phthiraptera), for example, Damalinia spp., Haematopinus spp., Linognathus spp., Pediculus spp., Trichodectes spp.

From the class of the Arachnida, for example, Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

From the class of the Bivalva, for example, Dreissena spp.

From the order of the Chilopoda, for example, Geophilus spp., Scutigera spp.

From the order of the Coleoptera, for example, Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

From the order of the Collembola, for example, Onychiurus armatus.

From the order of the Dermaptera, for example, Forficula auricularia.

From the order of the Diplopoda, for example, Blaniulus guttulatus.

From the order of the Diptera, for example, Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp., Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

From the class of the Gastropoda, for example, Arion spp., Biomphalaria spp., Bulinus spp., Deroceras spp., Galba spp., Lymnaea spp., Oncomelania spp., Succinea spp.

From the class of the helminths, for example, Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp.,

Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

It is furthermore possible to control protozoa, such as Eimeria.

From the order of the Heteroptera, for example, Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

From the order of the Homoptera, for example, Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

From the order of the Hymenoptera, for example, Diprion spp., Hoplocampa spp., Lasius spp., Monomorium

pharaonis, Vespa spp.

From the order of the Isopoda, for example, Armadillidium vulgare, Oniscus asellus, Porcellio scaber.

From the order of the Isoptera, for example, Reticulitermes spp., Odontotermes spp.

From the order of the Lepidoptera, for example, Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

From the order of the Orthoptera, for example, Acheta domesticus, Blatta orientalis, Blattella germanica, Gryllotalpa spp., Leucophaea maderae, Locusta spp., Melanoplus spp., Periplaneta americana, Schistocerca gregaria.

From the order of the Siphonaptera, for example, Ceratophyllus spp., Xenopsylla cheopis.

From the order of the Symphyla, for example, Scutigerella immaculata.

From the order of the Thysanoptera, for example, Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

From the order of the Thysanura, for example, Lepisma saccharina.

The phytoparasitic nematodes include, for example, Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

If appropriate, the compounds according to the invention can, at certain concentrations or application rates, also be used as herbicides, safeners, growth regulators or agents to improve plant properties, or as microbicides, for example as fungicides, antimycotics, bactericides, viricides (including agents against viroids) or as agents against MLO (Mycoplasma-like organisms) and RLO (Rickettsia-like organisms). If appropriate, they can also be employed as intermediates or precursors for the synthesis of other active compounds.

The active compounds can be converted to the customary formulations, such as solutions, emulsions, wettable powders, water- and oil-based suspensions, powders, dusts, pastes, soluble powders, soluble granules, granules for broadcasting, suspension-emulsion concentrates, natural materials impregnated with active compound, synthetic materials impregnated with active compound, fertilizers and microencapsulations in polymeric substances.

These formulations are produced in a known manner, for example by mixing the active compounds with extenders, that is liquid solvents and/or solid carriers, optionally with the use of surfactants, that is emulsifiers and/or dispersants and/or foam-formers. The formulations are prepared either in suitable plants or else before or during the application.

Suitable for use as auxiliaries are substances which are suitable for imparting to the composition itself and/or to preparations derived therefrom (for example spray liquors, seed dressings) particular properties such as certain technical properties and/or also particular biological properties. Typical suitable auxiliaries are: extenders, solvents and carriers.

Suitable extenders are, for example, water, polar and non-polar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as *N*-alkylpyrrolidones) and lactones, the sulphones and sulphoxides (such as dimethyl sulphoxide).

If the extender used is water, it is also possible to employ, for example, organic solvents as auxiliary solvents. Essentially, suitable liquid solvents are: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example petroleum fractions, mineral and vegetable oils, alcohols such as butanol or glycol and also their ethers and esters, ketones such as acetone, methyl ethyl ketone, methyl isobutyl ketone or cyclohexanone, strongly polar solvents such as dimethyl sulphoxide, and also water.

Suitable solid carriers are:
for example, ammonium salts and ground natural minerals such as kaolins, clays, talc, chalk, quartz, attapulgite, montmorillonite or diatomaceous earth, and ground synthetic minerals, such as finely divided silica, alumina and silicates; suitable solid carriers for granules are: for example, crushed and fractionated natural rocks such as calcite, marble, pumice,

sepiolite and dolomite, and also synthetic granules of inorganic and organic meals, and granules of organic material such as paper, sawdust, coconut shells, maize cobs and tobacco stalks; suitable emulsifiers and/or foam-formers are: for example, nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP-POE esters, alkyl aryl and/or POP-POE ethers, fat and/or POP-POE adducts, POE- and/or POP-polyol derivatives, POE- and/or POP-sorbitan- or -sugar adducts, alkyl or aryl sulphates, alkyl- or arylsulphonates and alkyl or aryl phosphates or the corresponding PO-ether adducts. Furthermore, suitable oligo- or polymers, for example those derived from vinylic monomers, from acrylic acid, from EO and/or PO alone or in combination with, for example, (poly)alcohols or (poly)amines. It is also possible to employ lignin and its sulphonic acid derivatives, unmodified and modified celluloses, aromatic and/or aliphatic sulphonic acids and their adducts with formaldehyde.

Tackifiers such as carboxymethylcellulose and natural and synthetic polymers in the form of powders, granules or latices, such as gum arabic, polyvinyl alcohol and polyvinyl acetate, as well as natural phospholipids such as cephalins and lecithins, and synthetic phospholipids, can be used in the formulations.

It is possible to use colorants such as inorganic pigments, for example iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

Other possible additives are perfumes, mineral or vegetable, optionally modified oils, waxes and nutrients (including trace nutrients), such as salts of iron, manganese, boron, copper, cobalt, molybdenum and zinc.

Stabilizers, such as low-temperature stabilizers, preservatives, antioxidants, light stabilizers or other agents which improve chemical and/or physical stability may also be present.

The formulations generally comprise between 0.01 and 98% by weight of active compound, preferably between 0.5 and 90%.

One skilled in the art will, of course, recognize that the formulation and mode of application of a toxicant may affect the activity of the material in a given application. Thus, for agricultural and general household pest use the present insecticidal compounds may be formulated as a granular of relatively large particle size (for example, 8/16 or 4/8 US Mesh), as water-soluble or water-dispersible granules, as powdery dusts, as wettable powders, as emulsifiable concentrates, as aqueous emulsions, as solutions, or as any of other known types of useful formulations, depending on the desired mode of application. It is to be understood that the amounts specified in this specification are intended to be approximate only, as if the word "about" were placed in front of the amounts specified.

These insecticidal compositions may be applied either as water-diluted sprays, or dusts, or granules to the areas in which suppression of insects is desired. These formulations may contain as little as 0.1 %, 0.2% or 0.5% to as much as 95% or more by weight of active ingredient.

Dusts are free flowing admixtures of the active ingredient with finely divided solids such as talc, natural clays, kieselguhr, flours such as walnut shell and cottonseed flours, and other organic and inorganic solids which act as dispersants and carriers for the toxicant; these finely divided solids have an average particle size of less than about 50 microns. A typical dust formulation useful herein is one containing 1.0 part or less of the insecticidal compound and 99.0 parts of talc.

Wettable powders, also useful formulations for insecticides, are in the form of finely divided particles that disperse readily in water or other dispersant. The wettable powder is ultimately applied to the locus where insect control is needed either as a dry dust or as an emulsion in water or other liquid. Typical carriers for wettable powders include Fuller's earth, kaolin clays, silicas, and other highly absorbent, readily wet inorganic diluents. Wettable powders normally are prepared to contain about 5-80% of active ingredient, depending on the absorbency of the carrier, and usually also contain a small amount of a wetting, dispersing or emulsifying agent to facilitate dispersion. For example, a useful wettable powder formulation contains 80.0 parts of the insecticidal compound, 17.9 parts of Palmetto clay, and 1.0 part of sodium lignosulfonate and 0.3 part of sulfonated aliphatic polyester as wetting agents. Additional wetting agents and/or oils will frequently be added to a tank mix for to facilitate dispersion on the foliage of the plant.

Other useful formulations for insecticidal applications are emulsifiable concentrates (ECs) which are homogeneous liquid compositions dispersible in water or other dispersant, and may consist entirely of the insecticidal compound and a liquid or solid emulsifying agent, or may also contain a liquid carrier, such as xylene, heavy aromatic naphthas, isophorone, or other non-volatile organic solvents. For insecticidal application these concentrates are dispersed in water or other liquid carrier and normally applied as a spray to the area to be treated. The percentage by weight of the essential active ingredient may vary according to the manner in which the composition is to be applied, but in general comprises 0.5 to 95% of active ingredient by weight of the insecticidal composition.

Flowable formulations are similar to ECs, except that the active ingredient is suspended in a liquid carrier, generally water. Flowables, like ECs, may include a small amount of a surfactant, and will typically contain active ingredients in the range of 0.5 to 95%, frequently from 10 to 50%, by weight of the composition. For application, flowables may be diluted in water or other liquid vehicle, and are normally applied as a spray to the area to be treated.

Typical wetting, dispersing or emulsifying agents used in agricultural formulations include, but are not limited to, the alkyl and alkylaryl sulfonates and sulfates and their sodium salts; alkylaryl polyether alcohols; sulfated higher alcohols; polyethylene oxides; sulfonated animal and vegetable oils; sulfonated petroleum oils; fatty acid esters of polyhydric alcohols and the ethylene oxide addition products of such esters; and the addition product of long-chain mercaptans and ethylene oxide. Many other types of useful surface-active agents are available in commerce. Surface-active agents, when used, normally comprise 1 to 15% by weight of the composition.

Other useful formulations include suspensions of the active ingredient in a relatively non-volatile solvent such as water, corn oil, kerosene, propylene glycol, or other suitable solvents.

Still other useful formulations for insecticidal applications include simple solutions of the active ingredient in a solvent in which it is completely soluble at the desired concentration, such as acetone, alkylated naphthalenes, xylene, or other organic solvents. Granular formulations, wherein the toxicant is carried on relative coarse particles, are of particular utility for aerial distribution or for penetration of cover crop canopy. Pressurized sprays, typically aerosols wherein the active ingredient is dispersed in finely divided form as a result of vaporization of a low-boiling dispersant solvent carrier may also be used. Water-soluble or water-dispersible granules are free flowing, non-dusty, and readily water-soluble or water-miscible. In use by the farmer on the field, the granular formulations, emulsifiable concentrates, flowable concentrates, aqueous emulsions, solutions, etc., may be diluted with water to give a concentration of active ingredient in the range of say 0.1 % or 0.2% to 1.5% or 2%.

The active compound according to the invention can be used in its commercially available formulations and in the use forms, prepared from these formulations, as a mixture with other active compounds, such as insecticides, attractants, sterilizing agents, bactericides, acaricides, nematicides, fungicides, growth-regulating substances, herbicides, safeners, fertilizers or semiochemicals.

When used as insecticides, the active compounds according to the invention can furthermore be present in their commercially available formulations and in the use forms, prepared from these formulations, as a mixture with synergists. Synergists are compounds which increase the action of the active compounds, without it being necessary for the synergistic agent added to be active itself.

When used as insecticides, the active compounds according to the invention can furthermore be present in their commercially available formulations and in the use forms, prepared from these formulations, as a mixture with inhibitors which reduce degradation of the active compound after use in the environment of the plant, on the surface of parts of plants or in plant tissues.

The active compound content of the use forms prepared from the commercially available formulations can vary within wide limits. The active compound concentration of the use forms can be from 0.00000001 to 95% by weight of active compound, preferably between 0.00001 and 1% by weight.

The compounds are employed in a customary manner appropriate for the use forms.

All plants and plant parts can be treated in accordance with the invention. Plants are to be understood as meaning in the present context all plants and plant populations such as desired and undesired wild plants or crop plants (including naturally occurring crop plants). Crop plants can be plants which can be obtained by conventional plant breeding and optimization methods or by biotechnological and genetic engineering methods or by combinations of these methods, including the transgenic plants and including the plant cultivars protectable or not protectable by plant breeders' rights. Plant parts are to be understood as meaning all parts and organs of plants above and below the ground, such as shoot, leaf, flower and root, examples which may be mentioned being leaves, needles, stalks, stems, flowers, fruit bodies, fruits, seeds, roots, tubers and rhizomes. The plant parts also include harvested material, and vegetative and generative propagation material, for example cuttings, tubers, rhizomes, offshoots and seeds.

Treatment according to the invention of the plants and plant parts with the active compounds is carried out directly or by allowing the compounds to act on the surroundings, habitat or storage space by the customary treatment methods, for example by immersion, spraying, evaporation, fogging, scattering, painting on, injection and, in the case of propagation material, in particular in the case of seeds, also by applying one or more coats.

Treatment according to the invention of the plants and plant parts with the active compound combinations is carried out directly or by allowing the compounds to act on the surroundings, habitat or storage space by the customary treatment methods, for example by immersion, spraying, evaporation, fogging, scattering, painting on, and, in the case of propagation material, in particular in the case of seeds, also by applying one or more coats.

The mixtures according to the invention are particularly suitable for treating seed. Here, the combinations according to the invention mentioned above as preferred or particularly preferred may be mentioned as being preferred. Thus, a large part of the damage to crop plants which is caused by pests occurs as early as when the seed is attacked during storage and after the seed is introduced into the soil, during and immediately after germination of the plants. This phase is particularly critical since the roots and shoots of the growing plant are particularly sensitive and even minor damage can lead to the death of the whole plant. Protecting the seed and the germinating plant by the use of suitable compositions is therefore of particularly great interest.

The control of pests by treating the seeds of plants has been known for a long time and is the subject of

continuous improvements. However, the treatment of seed entails a series of problems which cannot always be solved in a satisfactory manner. Thus, it is desirable to develop methods for protecting the seed and the germinating plant which dispense with the additional application of crop protection agents after sowing or after the emergence of the plants. It is furthermore desirable to optimize the amount of active compound employed in such a way as to provide maximum protection for the seed and the germinating plant from attack by pests, but without damaging the plant itself by the active compound employed. In particular, methods for the treatment of seed should also take into consideration the intrinsic insecticidal properties of transgenic plants in order to achieve optimum protection of the seed and the germinating plant with a minimum of crop protection agents being employed.

The present invention therefore in particular also relates to a method for the protection of seed and germinating plants from attack by pests, by treating the seed with a composition according to the invention. The invention likewise relates to the use of the compositions according to the invention for the treatment of seed for protecting the seed and the resultant plant from pests. Furthermore, the invention relates to seed which has been treated with a composition according to the invention so as to afford protection from pests.

One of the advantages of the present invention is that the particular systemic properties of the compositions according to the invention mean that treatment of the seed with these compositions not only protects the seed itself, but also the resulting plants after emergence, from pests. In this manner, the immediate treatment of the crop at the time of sowing or shortly thereafter can be dispensed with.

A further advantage is the synergistically increased insecticidal activity of the compositions according to the invention in comparison with the individual insecticidal active compound, which exceeds the anticipated activity of the two active compounds when applied individually. Also advantageous is the synergistically increased fungicidal activity of the compositions according to the invention in comparison with the individual fungicidal active compound, which exceeds the anticipated activity of the active compound when applied individually. This makes possible an optimization of the amount of active compound employed.

Furthermore, it must be considered as advantageous that the mixtures according to the invention can also be employed in particular in transgenic seed, the plants arising from this seed being capable of expressing a protein directed against pests. By treating such seed with the compositions according to the invention, certain pests can be controlled merely by the expression of the, for example, insecticidal protein, and additionally be protected by the compositions according to the invention against damage.

The compositions according to the invention are suitable for protecting seed of any plant variety as already mentioned above which is employed in agriculture, in the greenhouse, in forests or in horticulture. In particular, this takes the form of seed of maize, peanut, canola, oilseed rape, poppy, soya beans, cotton, beet (for example sugar beet and fodder beet), rice, sorghum and millet, wheat, barley, oats, rye, sunflower, tobacco, potatoes or vegetables (for example tomatoes, cabbage plants). The compositions according to the invention are likewise suitable for treating the seed of fruit plants and vegetables as already mentioned above. The treatment of the seed of maize, soya beans, cotton, wheat and canola or oilseed rape is of particular importance.

As already mentioned above, the treatment of transgenic seed with a composition according to the invention is also of particular importance. This takes the form of seed of plants which, as a rule, comprise at least one heterologous gene which governs the expression of a polypeptide with in particular insecticidal properties. In this context, the heterologous genes in transgenic seed may be derived from microorganisms such as Bacillus, Rhizobium, Pseudomonas, Serratia, Trichoderma, Clavibacter, Glomus or Gliocladium. The present invention is particularly suitable for the treatment of transgenic seed which comprises at least one heterologous gene orignating from Bacillus sp. and whose gene product shows activity against the European corn borer and/or the corn root worm. It is particularly preferably a heterologous gene derived from Bacillus thuringiensis.

In the context of the present invention, the composition according to the invention is applied to the seed either alone or in a suitable formulation. Preferably, the seed is treated in a state which is stable enough to avoid damage during treatment. In general, the seed may be treated at any point in time between harvest and sowing. The seed usually used has been separated from the plant and freed from cobs, shells, stalks, coats, hairs or the flesh of the fruits.

When treating the seed, care must generally be taken that the amount of the composition according to the invention applied to the seed and/or the amount of further additives is chosen in such a way that the germination of the seed is not adversely affected, or that the resulting plant is not damaged. This must be borne in mind in particular in the case of active compounds which may have phytotoxic effects at certain application rates.

As already mentioned above, it is possible to treat all plants and their parts according to the invention. In a preferred embodiment, wild plant species and plant cultivars, or those obtained by conventional biological breeding methods, such as crossing or protoplast fusion, and parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts", "parts of plants" and "plant parts" have been explained above.

Particularly preferably, plants of the plant cultivars which are in each case commercially available or in use are

treated according to the invention. Plant cultivars are to be understood as meaning plants having novel properties ("traits") which have been obtained by conventional breeding, by mutagenesis or by recombinant DNA techniques. These can be cultivars, bio- or genotypes.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the substances and compositions which can be used according to the invention, better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products are possible, which exceed the effects which were actually to be expected.

The transgenic plants or plant cultivars (obtained by genetic engineering) which are preferably to be treated according to the invention include all plants which, by virtue of the genetic modification, received genetic material which imparted particularly advantageous, useful traits to these plants. Examples of such traits are better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products. Further and particularly emphasized examples of such traits are a better defence of the plants against animal and microbial pests, such as against insects, mites, phytopathogenic fungi, bacteria and/or viruses, and also increased tolerance of the plants to certain herbicidally active compounds. Examples of transgenic plants which may be mentioned are the important crop plants, such as cereals (wheat, rice), maize, soya beans, potatoes, sugar beet, tomatoes, peas and other vegetable varieties, cotton, tobacco, oilseed rape and also fruit plants (with the fruits apples, pears, citrus fruits and grapes), and particular emphasis is given to maize, soya beans, potatoes, cotton, tobacco and oilseed rape. Traits that are emphasized are in particular increased defence of the plants against insects, arachnids, nematodes and slugs and snails by virtue of toxins formed in the plants, in particular those formed in the plants by the genetic material from Bacillus thuringiensis (for example by the genes CryIA(a), CryIA(b), CryIA(c), CryIIA, CryIIIA, CryIIIB2, Cry9c, Cry2Ab, Cry3Bb and CryIF and also combinations thereof) (referred to hereinbelow as "Bt plants"). Traits that are also particularly emphasized are the increased defence of the plants against fungi, bacteria and viruses by systemic acquired resistance (SAR), systemin, phytoalexins, elicitors and resistance genes and correspondingly expressed proteins and toxins. Traits that are furthermore particularly emphasized are the increased tolerance of the plants to certain herbicidally active compounds, for example imidazolinones, sulphonylureas, glyphosate or phosphinotricin (for example the "PAT" gene). The genes which impart the desired traits in question can also be present in combination with one another in the transgenic plants. Examples of "Bt plants" which may be mentioned are maize varieties, cotton varieties, soya bean varieties and potato varieties which are sold under the trade names YIELD GARD® (for example maize, cotton, soya beans), KnockOut® (for example maize), StarLink® (for example maize), Bollgard® (cotton), Nucotn® (cotton) and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize). Of course, these statements also apply to plant cultivars having these genetic traits or genetic traits still to be developed, which plant cultivars will be developed and/or marketed in the future.

The plants listed can be treated according to the invention in a particularly advantageous manner with the compounds of the general formula I and/or the active compound mixtures according to the invention. The preferred ranges stated above for the active compounds or mixtures also apply to the treatment of these plants. Particular emphasis is given to the treatment of plants with the compounds or mixtures specifically mentioned in the present text.

The active compounds according to the invention act not only against plant, hygiene and stored product pests, but also in the veterinary medicine sector against animal parasites (ecto- and endoparasites), such as hard ticks, soft ticks, mange mites, leaf mites, flies (biting and licking), parasitic fly larvae, lice, hair lice, feather lice and fleas. These parasites include:

From the order of the Anoplurida, for example, Haematopinus spp., Linognathus spp., Pediculus spp., Phtirus spp., Solenopotes spp.

From the order of the Mallophagida and the suborders Amblycerina and Ischnocerina, for example, Trimenopon spp., Menopon spp., Trinoton spp., Bovicola spp., Werneckiella spp., Lepikentron spp., Damalina spp., Trichodectes spp., Felicola spp.

From the order of the Diptera and the suborders Nematocerina and Brachycerina, for example, Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca

spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

From the order of the Siphonapterida, for example, Pulex spp., Ctenocephalides spp., Xenopsylla spp., Ceratophyllus spp.

From the order of the Heteropterida, for example, Cimex spp., Triatoma spp., Rhodnius spp., Panstrongylus spp.

From the order of the Blattarida, for example, Blatta orientalis, Periplaneta americana, Blattela germanica, Supella spp.

From the subclass of the Acari (Acarina) and the orders of the Meta- and Mesostigmata, for example, Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp.

From the order of the Actinedida (Prostigmata) and Acaridida (Astigmata), for example, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

The active compounds of the formula (I) according to the invention are also suitable for controlling arthropods which infest agricultural productive livestock, such as, for example, cattle, sheep, goats, horses, pigs, donkeys, camels, buffalo, rabbits, chickens, turkeys, ducks, geese and bees, other pets, such as, for example, dogs, cats, caged birds and aquarium fish, and also so-called test animals, such as, for example, hamsters, guinea pigs, rats and mice. By controlling these arthropods, cases of death and reduction in productivity (for meat, milk, wool, hides, eggs, honey etc.) should be diminished, so that more economic and easier animal husbandry is possible by use of the active compounds according to the invention.

The active compounds according to the invention are used in the veterinary sector and in animal husbandry in a known manner by enteral administration in the form of, for example, tablets, capsules, potions, drenches, granules, pastes, boluses, the feed-through process and suppositories, by parenteral administration, such as, for example, by injection (intramuscular, subcutaneous, intravenous, intraperitoneal and the like), implants, by nasal administration, by dermal use in the form, for example, of dipping or bathing, spraying, pouring on and spotting on, washing and powdering, and also with the aid of moulded articles containing the active compound, such as collars, ear marks, tail marks, limb bands, halters, marking devices and the like.

When used for cattle, poultry, pets and the like, the active compounds of the formula (I) can be used as formulations (for example powders, emulsions, free-flowing compositions), which comprise the active compounds in an amount of 1 to 80% by weight, directly or after 100 to 10 000-fold dilution, or they can be used as a chemical bath.

It has furthermore been found that the compounds according to the invention also have a strong insecticidal action against insects which destroy industrial materials.

The following insects may be mentioned as examples and as preferred - but without any limitation:

Beetles, such as Hylotrupes bajulus, Chlorophorus pilosis, Anobium punctatum, Xestobium rufovillosum, Ptilinus pecticomis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hymenopterons, such as Sirexjuvencus, Urocerus gigas, Urocerus gigas taignus, Urocerus augur;

Termites, such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Bristletails, such as Lepisma saccharina.

Industrial materials in the present connection are to be understood as meaning non-living materials, such as, preferably, plastics, adhesives, sizes, papers and cardboards, leather, wood and processed wood products and coating compositions.

The ready-to-use compositions may, if appropriate, comprise further insecticides and, if appropriate, one or more fungicides.

With respect to possible additional additives, reference may be made to the insecticides and fungicides mentioned above.

The compounds according to the invention can likewise be employed for protecting objects which come into contact with seawater or brackish water, such as hulls, screens, nets, buildings, moorings and signalling systems, against fouling.

Furthermore, the compounds according to the invention, alone or in combinations with other active compounds, may be employed as antifouling agents.

In domestic, hygiene and stored-product protection, the active compounds are also suitable for controlling

animal pests, in particular insects, arachnids and mites, which are found in enclosed spaces such as, for example, dwellings, factory halls, offices, vehicle cabins and the like. They can be employed alone or in combination with other active compounds and auxiliaries in domestic insecticide products for controlling these pests. They are active against sensitive and resistant species and against all developmental stages. These pests include:

From the order of the Scorpionidea, for example, Buthus occitanus.

From the order of the Acarina, for example, Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

From the order of the Araneae, for example, Aviculariidae, Araneidae.

From the order of the Opiliones, for example, Pseudoscorpiones chelifer, Pseudoscorpiones cheiridium, Opiliones phalangium.

From the order of the Isopoda, for example, Oniscus asellus, Porcellio scaber.

From the order of the Diplopoda, for example, Blaniulus guttulatus, Polydesmus spp.

From the order of the Chilopoda, for example, Geophilus spp.

From the order of the Zygentoma, for example, Ctenolepisma spp., Lepisma saccharina, Lepismodes inquilinus.

From the order of the Blattaria, for example, Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

From the order of the Saltatoria, for example, Acheta domesticus.

From the order of the Dermaptera, for example, Forficula auricularia.

From the order of the Isoptera, for example, Kalotermes spp., Reticulitermes spp.

From the order of the Psocoptera, for example, Lepinatus spp., Liposcelis spp.

From the order of the Coleoptera, for example, Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

From the order of the Diptera, for example, Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga camaria, Simulium spp., Stomoxys calcitrans, Tipula paludosa.

From the order of the Lepidoptera, for example, Achroia grisella, Galleria mellonella, Plodia interpunctella, Tinea cloacella, Tinea pellionella, Tineola bisselliella.

From the order of the Siphonaptera, for example, Ctenocephalides canis, Ctenocephalides felis, Pulex irritans, Tunga penetrans, Xenopsylla cheopis.

From the order of the Hymenoptera, for example, Camponotus herculeanus, Lasius fuliginosus, Lasius niger, Lasius umbratus, Monomorium pharaonis, Paravespula spp., Tetramorium caespitum.

From the order of the Anoplura, for example, Pediculus humanus capitis, Pediculus humanus corporis, Pemphigus spp., Phylloera vastatrix, Phthirus pubis.

From the order of the Heteroptera, for example, Cimex hemipterus, Cimex lectularius, Rhodinus prolixus, Triatoma infestans.

In the field of household insecticides, they are used alone or in combination with other suitable active compounds, such as phosphoric esters, carbamates, pyrethroids, neonicotinoids, growth regulators or active compounds from other known classes of insecticides.

They are used in aerosols, pressure-free spray products, for example pump and atomizer sprays, automatic fogging systems, foggers, foams, gels, evaporator products with evaporator tablets made of cellulose or polymer, liquid evaporators, gel and membrane evaporators, propeller-driven evaporators, energy-free, or passive, evaporation systems, moth papers, moth bags and moth gels, as granules or dusts, in baits for spreading or in bait stations.

Soil conditioners are materials which, when added to the soil, promote a variety of benefits for the efficacious growth of plants. Soil conditioners are used to reduce soil compaction, promote and increase effectiveness of drainage, improve soil permeability, promote optimum plant nutrient content in the soil, and promote better pesticide and fertilizer incorporation. When the active insecticidal compounds of the present invention are used in combination with one or more of second compounds, e.g., with other materials such as soil conditioners, the soil conditioners include organic matter, such as humus, which promotes retention of cation plant nutrients in the soil; mixtures of cation nutrients, such as calcium, magnesium, potash, sodium, and hydrogen complexes; or microorganism compositions which promote conditions in the soil favorable to plant growth. Such microorganism compositions include, for example, *bacillus, pseudomonas, azotobacter, azospirillum, rhizobium,* and soil-borne *cyanobacteria.*

Fertilizers are plant food supplements, which commonly contain nitrogen, phosphorus, and potassium. When the active insecticidal compounds of the present invention are used in combination with one or more of second compounds, e.g., with other materials such as fertilizers, the fertilizers include nitrogen fertilizers, such as ammonium sulfate,

ammonium nitrate, and bone meal; phosphate fertilizers, such as superphosphate, triple superphosphate, ammonium sulfate, and diammonium sulfate; and potassium fertilizers, such as muriate of potash, potassium sulfate, and potassium nitrate, and other fertilizers.

Preparation and use of the active compounds according to the invention are illustrated in the examples below.

**Preparation Examples:**

**EXAMPLE 1**

Preparation of 1-[(1S)-2,3-dihydro-1H-inden-1-yl]-3-(2-hydroxyethyl)thiourea

(Cmpd. No 1-2)

A stirred mixture of 550 mg (3,14 mmol) (S)-(+)-indanylisothiocanate (commercial available) and 191 mg (3,14 mmol) 2-aminoethanol in 9 mL toluene was warmed to 60 ˚C for 4 h, than allowed to cool to ambient temperature and concentrated under reduced pressure to a residue. The residue was purified by chromatography (silica gel, $CH_2Cl_2$/acetonitrile = vol. 7:3) to afford 700 mg 1-[(1S)-2,3-dihydro-1H-inden-1-yl]-3-(2-hydroxyethyl)thiourea as a colourless oil. The NMR spectrum was consistent with the proposed structure.
logP[1)] = 1,65

**EXAMPLE 2**

Preparation of 1-(2,3-dihydro-1H-inden-1-yl)-3-(2-methoxyethyl)thiourea

(Cmpd. No 1-4)

A stirred mixture of 300 mg (2,25 mmol) 1-aminoindane (commercial available) and 277 mg (2,36 mmol) 2-methoxy-ethylisothiocyanate in 6 mL toluene was warmed to 60 ˚C for 4 h, than allowed to cool to ambient temperature and concentrated under reduced pressure to a residue. The residue was purified by chromatography (silica gel, cyclohexane/ethyl acetate = vol. 2:1) to afford 500 mg 1-(2,3-dihydro-1H-inden-1-yl)-3-(2-methoxyethyl)thiourea as a colourless solid. The NMR spectrum was consistent with the proposed structure.
logP[1)] = 2,07

**EXAMPLE 3**

Preparation of 1-(4-fluoro-2,3-dihydro-1H-inden-l-yl)-3-(2-hydroxyethyl)thiourea

(Cmpd. No. 1-14)

A stirred mixture of 600 mg (3,4 mmol) 4-fluoro-1-aminoindane and 778 mg (4.37 mmol) 1,1-thiocarbonyldiimidazole in dichloromethane was kept at ambient temperature for 1,5 h. After addition of 230 mg (3.74 mmol) 2-aminoethanol dissolved in 3 mL dichloromethane the resulting mixture was stirred for further 72 h at ambient temperature. Then, the mixture was diluted with ethyl acetate and washed with 1 N HCl and saturated $NaHCO_3$ solution. After phase separation, the organic phase was dried with solid $Na_2SO_4$, filtered and evaporated under reduced pressure. The residue was purified by chromatography (silica gel, cyclohexane/ethyl acetate = vol. 5:1) to afford 350 mg 1-(4-fluoro-2,3-dihydro-1H-inden-1-yl)-3-(2-hydroxyethyl)thiourea. The NMR spectrum was consistent with the proposed structure.

$$\log P^{1)} = 1{,}74$$

[1)] logP (HCOOH) determined according to EEC-Directive 79/831, Annex V.A8 using HPLC (gradient method, acetonitrile/0.1% HCOOH)

The following tables set forth additional hydroxyethyl thiourea derivatives of formula (I)

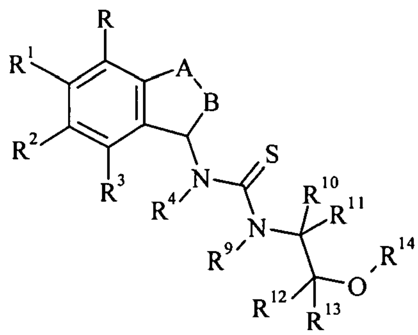

(I)

**Table 1**

Compounds of formula (I-1) wherein A is $-CHR^5-$, B is $-CHR^8-$ and $R^4$ and $R^{11}$ to $R^{13}$ are hydrogen:

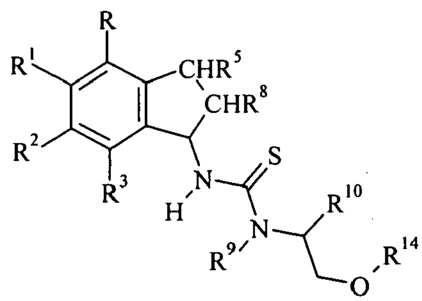

I-1

| Cmpd No. | R | $R^1$ | $R^2$ | $R^3$ | $R^5$ | $R^8$ | $R^9$ | $R^{10}$ | $R^{14}$ | Phys. Data[1)] |
|---|---|---|---|---|---|---|---|---|---|---|
| 1-1 | H | H | H | H | H | H | H | H | H | |
| 1-2* | H | H | H | H | H | H | H | H | H | 1,56 |
| 1-3** | H | H | H | H | H | H | H | H | H | 1,65 |
| 1-4 | H | H | H | H | H | H | H | H | $CH_3$ | 2,07 |
| 1-5* | H | H | H | H | H | H | H | H | $CH_3$ | |
| 1-6** | H | H | H | H | H | H | H | H | $CH_3$ | |
| 1-7 | H | H | H | H | H | $CH_3$ | H | H | H | |
| 1-8 | H | H | H | H | H | $CH_3^+$ | H | H | H | |
| 1-9 | H | H | H | H | H | $CH_3^{++}$ | H | H | H | |
| 1-10* | H | H | H | H | H | $CH_3^+$ | H | H | H | |
| 1-11* | H | H | H | H | H | $CH_3^{++}$ | H | H | H | |
| 1-12** | H | H | H | H | H | $CH_3^+$ | H | H | H | |
| 1-13** | H | H | H | H | H | $CH_3^{++}$ | H | H | H | |
| 1-14 | F | H | H | H | H | H | H | H | H | 1,74 |

(continued)

| Cmpd No. | R | $R^1$ | $R^2$ | $R^3$ | $R^5$ | $R^8$ | $R^9$ | $R^{10}$ | $R^{14}$ | Phys. Data[1)] |
|---|---|---|---|---|---|---|---|---|---|---|
| 1-15* | F | H | H | H | H | H | H | H | H | |
| 1-16** | F | H | H | H | H | H | H | H | H | |
| 1-17 | F | H | H | H | H | H | H | H | $CH_3$ | 2,21 |
| 1-18* | F | H | H | H | H | H | H | H | $CH_3$ | |
| 1-19** | F | H | H | H | H | H | H | H | $CH_3$ | |
| 1-20 | H | H | H | H | H | H | H | $CH_3$ | H | |
| 1-21 * | H | H | H | H | H | H | H | $CH_3$ | H | 1,78 |
| 1-22* | H | H | H | H | H | H | H | $CH_3$ | H | |
| 1-23 | H | H | H | H | H | H | H | H | *t*Bu | |
| 1-24* | H | H | H | H | H | H | H | H | *t*Bu | |
| 1-25** | H | H | H | H | H | H | H | H | *t*Bu | |
| 1-26 | H | H | H | H | H | H | H | H | $COCH_3$ | |
| 1-27* | H | H | H | H | H | H | H | H | $COCH_3$ | |
| 1-28** | H | H | H | H | H | H | H | H | $COCH_3$ | |
| 1-29 | H | H | H | H | H | H | H | H | COEt | |
| 1-30* | H | H | H | H | H | H | H | H | COEt | |
| 1-31* | H | H | H | H | H | H | H | H | COEt | |
| 1-32 | H | H | H | H | H | H | H | H | COiPr | |
| 1-33* | H | H | H | H | H | H | H | H | COiPr | |
| 1-34** | H | H | H | H | H | H | H | H | COiPr | |
| 1-35 | H | H | H | H | H | H | H | H | Et | |
| 1-36* | H | H | H | H | H | H | H | H | Et | |
| 1-37** | H | H | H | H | H | H | H | H | Et | |
| 1-38 | H | H | H | H | H | H | H | H | CO*t*Bu | |
| 1-39* | H | H | H | H | H | H | H | H | CO*t*Bu | |
| 1-40** | H | H | H | H | H | H | H | H | CO*t*Bu | |
| 1-41 | H | H | H | H | H | H | H | H | *i*Pr | |
| 1-42* | H | H | H | H | H | H | H | H | *i*Pr | |
| 1-43** | H | H | H | H | H | H | H | H | *i*Pr | |
| 1-44 | H | H | H | H | H | H | H | H | COO*t*Bu | |
| 1-45* | H | H | H | H | H | H | H | H | COO*t*Bu | |
| 1-46** | H | H | H | H | H | H | H | H | COO*t*Bu | |
| 1-47 | F | H | H | H | H | H | H | H | tBu | |
| 1-48* | F | H | H | H | H | H | H | H | tBu | |
| 1-49** | F | H | H | H | H | H | H | H | tBu | |
| 1-50 | F | H | H | H | H | H | H | H | COCH3 | |
| 1-51* | F | H | H | H | H | H | H | H | COCH3 | |

(continued)

| Cmpd No. | R | $R^1$ | $R^2$ | $R^3$ | $R^5$ | $R^8$ | $R^9$ | $R^{10}$ | $R^{14}$ | Phys. Data1) |
|---|---|---|---|---|---|---|---|---|---|---|
| 1-52** | F | H | H | H | H | H | H | H | COCH3 | |
| 1-53 | F | H | H | H | H | H | H | H | COEt | |
| 1-54* | F | H | H | H | H | H | H | H | COEt | |
| 1-55* | F | H | H | H | H | H | H | H | COEt | |
| 1-56 | F | H | H | H | H | H | H | H | COiPr | |
| 1-57* | F | H | H | H | H | H | H | H | COiPr | |
| 1-58** | F | H | H | H | H | H | H | H | COiPr | |
| 1-59 | F | H | H | H | H | H | H | H | Et | |
| 1-60* | F | H | H | H | H | H | H | H | Et | |
| 1-61** | F | H | H | H | H | H | H | H | Et | |
| 1-62 | F | H | H | H | H | H | H | H | COtBu | |
| 1-63 * | F | H | H | H | H | H | H | H | COtBu | |
| 1-64** | F | H | H | H | H | H | H | H | COtBu | |
| 1-65 | F | H | H | H | H | H | H | H | iPr | |
| 1-66* | F | H | H | H | H | H | H | H | iPr | |
| 1-67** | F | H | H | H | H | H | H | H | iPr | |
| 1-68 | F | H | H | H | H | H | H | H | COOtBu | |
| 1-69* | F | H | H | H | H | H | H | H | COOtBu | |
| 1-70** | F | H | H | H | H | H | H | H | COOtBu | |
| 1-71 | Cl | H | H | H | H | H | H | H | H | |
| 1-72 * | Cl | H | H | H | H | H | H | H | H | |
| 1-73** | Cl | H | H | H | H | H | H | H | H | |
| 1-74 | $CH_3$ | H | H | H | H | H | H | H | H | |
| 1-75* | $CH_3$ | H | H | H | H | H | H | H | H | |
| 1-76* | $CH_3$ | H | H | H | H | H | H | H | H | |
| 1-77 | Br | H | H | H | H | H | H | H | H | |
| 1-78 | $CF_3$ | H | H | H | H | H | H | H | H | |
| 1-79 | $OCH_3$ | H | H | H | H | H | H | H | H | |
| 1-80 | F | H | F | H | H | H | H | H | H | |
| 1-81* | F | H | F | H | H | H | H | H | H | |
| 1-82** | F | H | F | H | H | H | H | H | H | |
| 1-83 | F | H | H | F | H | H | H | H | H | 1,68 |
| 1-84* | F | H | H | F | H | H | H | H | H | |
| 1-85** | F | H | H | F | H | H | H | H | H | |
| 1-86 | Cl | H | F | H | H | H | H | H | H | |
| 1-87* | Cl | H | F | H | H | H | H | H | H | |
| 1-88** | Cl | H | F | H | H | H | H | H | H | |

(continued)

| Cmpd No. | R | $R^1$ | $R^2$ | $R^3$ | $R^5$ | $R^8$ | $R^9$ | $R^{10}$ | $R^{14}$ | Phys. Data[1)] |
|---|---|---|---|---|---|---|---|---|---|---|
| 1-89 | Cl | H | H | F | H | H | H | H | H | |
| 1-90* | Cl | H | H | F | H | H | H | H | H | |
| 1-91** | Cl | H | H | F | H | H | H | H | H | |
| 1-92 | Cl | H | Cl | H | H | H | H | H | H | |
| 1-93* | Cl | H | Cl | H | H | H | H | H | H | |
| 1-94** | Cl | H | Cl | H | H | H | H | H | H | |
| 1-95 | Cl | H | H | Cl | H | H | H | H | H | |
| 1-96* | Cl | H | H | Cl | H | H | H | H | H | |
| 1-97** | Cl | H | H | Cl | H | H | H | H | H | |
| 1-98 | $CH_3$ | H | F | H | H | H | H | H | H | |
| 1-99* | $CH_3$ | H | F | H | H | H | H | H | H | |
| 1-100** | $CH_3$ | H | F | H | H | H | H | H | H | |
| 1-101 | $CH_3$ | H | H | F | H | H | H | H | H | |
| 1-102* | $CH_3$ | H | H | F | H | H | H | H | H | |
| 1-103** | $CH_3$ | H | H | F | H | H | H | H | H | |
| 1-104 | H | H | H | H | H | H | 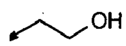 | H | H | |
| 1-105* | H | H | H | H | H | H | 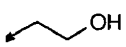 | H | H | 1,64 |
| 1-106** | H | H | H | H | H | H | 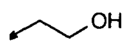 | H | H | |
| 1-107* | H | H | H | H | H | H | H | H | $CH(CF_3)_2$ | |
| 1-108* | H | H | H | H | H | H | H | H | $CH=CH_2$ | |
| 1-109* | H | H | H | H | H | H | H | H | $CH_2CH_2OH$ | |
| 1-110* | H | H | H | H | H | H | H | H | $CF_3$ | |

Here and in the following tables:
*(1S)-amino isomer + (2S) - methyl isomer
**(1R)-amino isomer ++ (2R) - methyl isomer

**Table 2**

Compounds of formula (I) wherein A is -O -, B is -$CHR^8$- and $R^4$ and $R^{11}$ to $R^{13}$ are hydrogen:

(continued)

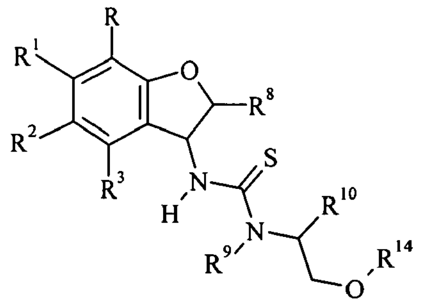

I-2

| Cmpd No. | R | $R^1$ | $R^2$ | $R^3$ | $R^8$ | $R^9$ | $R^{10}$ | $R^{14}$ | Phys. Data[1)] |
|---|---|---|---|---|---|---|---|---|---|
| 2-1 | H | H | H | H | H | H | H | H | |
| 2-2* | H | H | H | H | H | H | H | H | |
| 2-3** | H | H | H | H | H | H | H | H | |
| 2-4 | F | H | H | H | H | H | H | H | |
| 2-5 | F | H | F | H | H | H | H | H | |
| 2-6 | F | H | H | F | H | H | H | H | |
| 2-7 | Cl | H | H | H | H | H | H | H | |
| 2-8 | Cl | H | Cl | H | H | H | H | H | |
| 2-9 | Cl | H | H | Cl | H | H | H | H | |
| 2-10 | Cl | H | F | H | H | H | H | H | |
| 2-11 | Cl | H | H | F | H | H | H | H | |
| 2-12 | $CH_3$ | H | H | H | H | H | H | H | |
| 2-13 | $CH_3$ | H | F | H | H | H | H | H | |
| 2-14 | $CH_3$ | H | H | F | H | H | H | H | |
| 2-15 | Br | H | H | H | H | H | H | H | |
| 2-16 | $OCH_3$ | H | H | H | H | H | H | H | |
| 2-17 | $CF_3$ | H | H | H | H | H | H | H | |

**Table 3**

| |
|---|
| Compounds of formula (I) wherein A is $-OCHR^5-$, B is $-CHR^8-$ and $R^4$ and $R^{11}$ to $R^{13}$ are hydrogen: |

(continued)

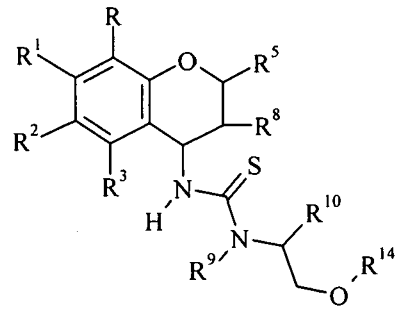

I-3

| Cmpd No. | R | $R^1$ | $R^2$ | $R^3$ | $R^5$ | $R^8$ | $R^9$ | $R^{10}$ | $R^{14}$ | Phys. Data[1)] |
|---|---|---|---|---|---|---|---|---|---|---|
| 3-1 | H | H | H | H | H | H | H | H | H | 1,42 |
| 3-2* | H | H | H | H | H | H | H | H | H | |
| 3-3** | H | H | H | H | H | H | H | H | H | |
| 3-4 | F | H | H | H | H | H | H | H | H | |
| 3-5 | F | H | F | H | H | H | H | H | H | |
| 3-6 | F | H | H | F | H | H | H | H | H | |
| 3-7 | Cl | H | H | H | H | H | H | H | H | |
| 3-8 | Cl | H | Cl | H | H | H | H | H | H | |
| 3-9 | Cl | H | H | Cl | H | H | H | H | H | |
| 3-10 | Cl | H | F | H | H | H | H | H | H | |
| 3-11 | Cl | H | H | F | H | H | H | H | H | |
| 3-12 | $CH_3$ | H | H | H | H | H | H | H | H | |
| 3-13 | $CH_3$ | H | F | H | H | H | H | H | H | |
| 3-14 | $CH_3$ | H | H | F | H | H | H | H | H | |
| 3-15 | Br | H | H | H | H | H | H | H | H | |
| 3-16 | $OCH_3$ | H | H | H | H | H | H | H | H | |
| 3-17 | $CF_3$ | H | H | H | H | H | H | H | H | |

**Table 4**

Compounds of formula (I) wherein A is $-CHR^5CHR^6-$, B is $-CHR^8-$ and, $R^4$, $R^5$, $R^6$, $R^8$ and $R^{11}$-$R^{13}$ are hydrogen:

(continued)

| 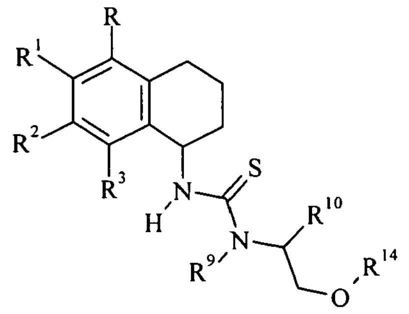 | | | | | | | | |
|---|---|---|---|---|---|---|---|---|
| I-4 | | | | | | | | |
| Cmpd No. | R | $R^1$ | $R^2$ | $R^3$ | $R^9$ | $R^{10}$ | $R^{14}$ | Phys. Data[1)] |
| 4-1 | H | H | H | H | H | H | H | |
| 4-2 * | H | H | H | H | H | H | H | 1,79 |
| 4-3** | H | H | H | H | H | H | H | |
| 4-4 | F | H | H | H | H | H | H | |
| 4-5 | F | H | F | H | H | H | H | |
| 4-6 | F | H | H | F | H | H | H | |
| 4-7 | Cl | H | H | H | H | H | H | |
| 4-8 | Cl | H | Cl | H | H | H | H | |
| 4-9 | Cl | H | H | Cl | H | H | H | |
| 4-10 | Cl | H | F | H | H | H | H | |
| 4-11 | Cl | H | H | F | H | H | H | |
| 4-12 | $CH_3$ | H | H | H | H | H | H | |
| 4-13 | $CH_3$ | H | F | H | H | H | H | |
| 4-14 | $CH_3$ | H | H | F | H | H | H | |
| 4-15 | Br | H | H | H | H | H | H | |
| 4-16 | $OCH_3$ | H | H | H | H | H | H | |
| 4-17 | $CF_3$ | H | H | H | H | H | H | |

**Table 5**

| Compounds of formula (I) wherein A is $S(O)_nCHR^5$-, B is $-CHR^8$-, n is 0 and $R^4$, $R^5$, $R^8$ and R''-$R^{13}$ are hydrogen: |
|---|

(continued)

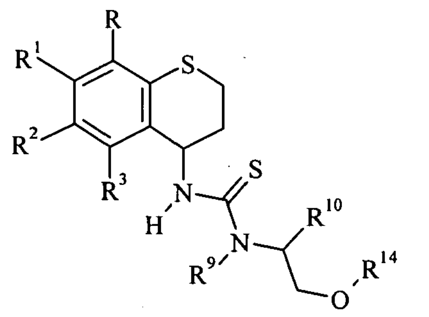

I-5

| Cmpd No. | R | $R^1$ | $R^2$ | $R^3$ | $R^9$ | $R^{10}$ | $R^{14}$ | Phys. Data[1)] |
|---|---|---|---|---|---|---|---|---|
| 5-1 | H | H | H | H | H | H | H | |
| 5-2* | H | H | H | H | H | H | H | |
| 5-3** | H | H | H | H | H | H | H | |
| 5-4 | F | H | H | H | H | H | H | |
| 5-5 | F | H | F | H | H | H | H | |
| 5-6 | F | H | H | F | H | H | H | |
| 5-7 | Cl | H | H | H | H | H | H | |
| 5-8 | Cl | H | Cl | H | H | H | H | |
| 5-9 | Cl | H | H | Cl | H | H | H | |
| 5-10 | Cl | H | F | H | H | H | H | |
| 5-11 | Cl | H | H | F | H | H | H | |
| 5-12 | $CH_3$ | H | H | H | H | H | H | |
| 5-13 | $CH_3$ | H | F | H | H | H | H | |
| 5-14 | $CH_3$ | H | H | F | H | H | H | |
| 5-15 | Br | H | H | H | H | H | H | |
| 5-16 | $OCH_3$ | H | H | H | H | H | H | |
| 5-17 | $CF_3$ | H | H | H | H | H | H | |

logP (HCOOH) determined according to EEC-Directive 79/831, Annex V.A8 using HPLC (gradient method, acetonitrile/ 0.1% HCOOH)

The following tables are examples for isothiocyanate intermediates of formula (II-a)

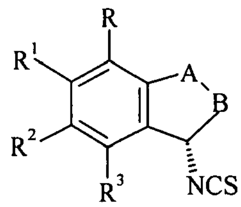

(II-a)

**Table 6**

Compounds of formula (II-a) wherein A is $-CHR^5-$ and B is $-CHR^8-$:

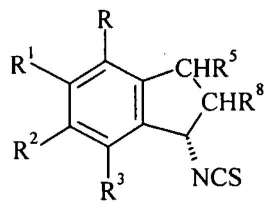

II-a1

| Cmpd No. | R | $R^1$ | $R^2$ | $R^3$ | $R^5$ | $R^8$ | Phys. Data[1)] |
|---|---|---|---|---|---|---|---|
| 6-1 | F | H | H | H | H | H | |
| 6-2 | F | H | F | H | H | H | |
| 6-3 | F | H | H | F | H | H | |
| 6-4 | F | F | H | H | H | H | |
| 6-5 | F | Cl | H | H | H | H | |
| 6-6 | F | H | Cl | H | H | H | |
| 6-7 | F | H | H | Cl | H | H | |
| 6-8 | Cl | H | H | H | H | H | |
| 6-9 | H | F | H | H | H | H | |
| 6-10 | H | Cl | H | H | H | H | |
| 6-11 | H | H | F | H | H | H | |
| 6-12 | H | H | Cl | H | H | H | |
| 6-13 | H | H | H | F | H | H | |
| 6-14 | H | H | H | Cl | H | H | |
| 6-15 | Cl | F | H | H | H | H | |
| 6-16 | Cl | H | F | H | H | H | |
| 6-17 | Cl | H | H | F | H | H | |
| 6-18 | F | $CH_3$ | H | H | H | H | |
| 6-19 | F | H | $CH_3$ | H | H | H | |

(continued)

| Cmpd No. | R | $R^1$ | $R^2$ | $R^3$ | $R^5$ | $R^8$ | Phys. Data[1)] |
|---|---|---|---|---|---|---|---|
| 6-20 | F | H | H | $CH_3$ | H | H | |
| 6-21 | $CH_3$ | F | H | H | H | H | |
| 6-22 | $CH_3$ | H | F | H | H | H | |
| 6-23 | $CH_3$ | H | H | F | H | H | |
| 6-24 | Cl | Cl | H | H | H | H | |
| 6-25 | Cl | H | Cl | H | H | H | |
| 6-26 | Cl | H | H | Cl | H | H | |

**Table 7**

Compounds of formula (II-a) wherein A is -O- and B is -CHR[8]-:

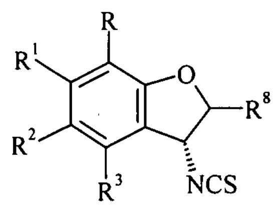

II-a2

| Cmpd No. | R | $R^1$ | $R^2$ | $R^3$ | $R^8$ | Phys. Data[1)] |
|---|---|---|---|---|---|---|
| 7-1 | F | H | H | H | H | |
| 7-2 | F | H | F | H | H | |
| 7-3 | F | H | H | F | H | |
| 7-4 | F | F | H | H | H | |
| 7-5 | F | Cl | H | H | H | |
| 7-6 | F | H | Cl | H | H | |
| 7-7 | F | H | H | Cl | H | |
| 7-8 | Cl | H | H | H | H | |
| 7-9 | H | F | H | H | H | |
| 7-10 | H | Cl | H | H | H | |
| 7-11 | H | H | F | H | H | |
| 7-12 | H | H | Cl | H | H | |
| 7-13 | H | H | H | F | H | |
| 7-14 | H | H | H | Cl | H | |
| 7-15 | Cl | F | H | H | H | |
| 7-16 | Cl | H | F | H | H | |
| 7-17 | Cl | H | H | F | H | |
| 7-18 | F | $CH_3$ | H | H | H | |

(continued)

| Cmpd No. | R | $R^1$ | $R^2$ | $R^3$ | $R^8$ | Phys. Data[1)] |
|---|---|---|---|---|---|---|
| 7-19 | F | H | $CH_3$ | H | H | |
| 7-20 | F | H | H | $CH_3$ | H | |
| 7-21 | $CH_3$ | F | H | H | H | |
| 7-22 | $CH_3$ | H | F | H | H | |
| 7-23 | $CH_3$ | H | H | F | H | |
| 7-24 | Cl | Cl | H | H | H | |
| 7-25 | Cl | H | Cl | H | H | |
| 7-26 | Cl | H | H | Cl | H | |

**Table 8**

Compounds of formula (II-a) wherein A is $-OCHR^5-$ and B is $-CHR^8-$:

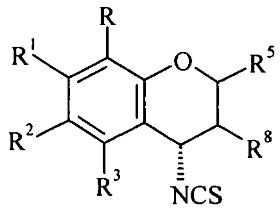

II-a3

| Cmpd No. | R | $R^1$ | $R^2$ | $R^3$ | $R^5$ | $R^8$ | Phys. Data[1)] |
|---|---|---|---|---|---|---|---|
| 8-1 | F | H | H | H | H | H | |
| 8-2 | F | H | F | H | H | H | |
| 8-3 | F | H | H | F | H | H | |
| 8-4 | F | F | H | H | H | H | |
| 8-5 | F | Cl | H | H | H | H | |
| 8-6 | F | H | Cl | H | H | H | |
| 8-7 | F | H | H | Cl | H | H | |
| 8-8 | Cl | H | H | H | H | H | |
| 8-9 | H | F | H | H | H | H | |
| 8-10 | H | Cl | H | H | H | H | |
| 8-11 | H | H | F | H | H | H | |
| 8-12 | H | H | Cl | H | H | H | |
| 8-13 | H | H | H | F | H | H | |
| 8-14 | H | H | H | Cl | H | H | |
| 8-15 | Cl | F | H | H | H | H | |
| 8-16 | Cl | H | F | H | H | H | |
| 8-17 | Cl | H | H | F | H | H | |

(continued)

| Cmpd No. | R | $R^1$ | $R^2$ | $R^3$ | $R^5$ | $R^8$ | Phys. Data[1)] |
|---|---|---|---|---|---|---|---|
| 8-18 | F | $CH_3$ | H | H | H | H | |
| 8-19 | F | H | $CH_3$ | H | H | H | |
| 8-20 | F | H | H | $CH_3$ | H | H | |
| 8-21 | $CH_3$ | F | H | H | H | H | |
| 8-22 | $CH_3$ | H | F | H | H | H | |
| 8-23 | $CH_3$ | H | H | F | H | H | |
| 8-24 | Cl | Cl | H | H | H | H | |
| 8-25 | Cl | H | Cl | H | H | H | |
| 8-26 | Cl | H | H | Cl | H | H | |

**Table 9**

Compounds of formula (II-a) wherein A is $-CHR^5CHR^6-$ and B is $-CHR^8-$, $R^5$,$R^6$ and $R^8$ are hydrogen:

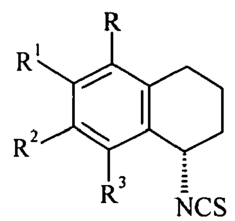

II-a4

| Cmpd No. | R | $R^1$ | $R^2$ | $R^3$ | Phys. Data[1)] |
|---|---|---|---|---|---|
| 9-1 | F | H | H | H | |
| 9-2 | F | H | F | H | |
| 9-3 | F | H | H | F | |
| 9-4 | F | F | H | H | |
| 9-5 | F | Cl | H | H | |
| 9-6 | F | H | Cl | H | |
| 9-7 | F | H | H | Cl | |
| 9-8 | Cl | H | H | H | |
| 9-9 | H | F | H | H | |
| 9-10 | H | Cl | H | H | |
| 9-11 | H | H | F | H | |
| 9-12 | H | H | Cl | H | |
| 9-13 | H | H | H | F | |
| 9-14 | H | H | H | Cl | |
| 9-15 | Cl | F | H | H | |
| 9-16 | Cl | H | F | H | |

(continued)

| Cmpd No. | R | $R^1$ | $R^2$ | $R^3$ | Phys. Data[1)] |
|---|---|---|---|---|---|
| 9-17 | Cl | H | H | F | |
| 9-18 | F | $CH_3$ | H | H | |
| 9-19 | F | H | $CH_3$ | H | |
| 9-20 | F | H | H | $CH_3$ | |
| 9-21 | $CH_3$ | F | H | H | |
| 9-22 | $CH_3$ | H | F | H | |
| 9-23 | $CH_3$ | H | H | F | |
| 9-24 | Cl | Cl | H | H | |
| 9-25 | Cl | H | Cl | H | |
| 9-26 | Cl | H | H | Cl | |

**Table 10**

Compounds of formula (II-a) wherein A is $S(O)_nCHR^5$- and B is $-CHR^8$, n is 0, $R^5$ and $R^8$ are hydrogen:

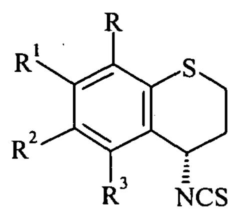

II-a5

| Cmpd No. | R | $R^1$ | $R^2$ | $R^3$ | Phys. Data[1)] |
|---|---|---|---|---|---|
| 10-1 | F | H | H | H | |
| 10-2 | F | H | F | H | |
| 10-3 | F | H | H | F | |
| 10-4 | F | F | H | H | |
| 10-5 | F | Cl | H | H | |
| 10-6 | F | H | Cl | H | |
| 10-7 | F | H | H | Cl | |
| 10-8 | Cl | H | H | H | |
| 10-9 | H | F | H | H | |
| 10-10 | H | Cl | H | H | |
| 10-11 | H | H | F | H | |
| 10-12 | H | H | Cl | H | |
| 10-13 | H | H | H | F | |
| 10-14 | H | H | H | Cl | |
| 10-15 | Cl | F | H | H | |

(continued)

| Cmpd No. | R | $R^1$ | $R^2$ | $R^3$ | Phys. Data1) |
|---|---|---|---|---|---|
| 10-16 | Cl | H | F | H | |
| 10-17 | Cl | H | H | F | |
| 10-18 | F | $CH_3$ | H | H | |
| 10-19 | F | H | $CH_3$ | H | |
| 10-20 | F | H | H | $CH_3$ | |
| 10-21 | $CH_3$ | F | H | H | |
| 10-22 | $CH_3$ | H | F | H | |
| 10-23 | $CH_3$ | H | H | F | |
| 10-24 | Cl | Cl | H | H | |
| 10-25 | Cl | H | Cl | H | |
| 10-26 | Cl | H | H | Cl | |
| 1) 1H-NMR (d6-DMSO) characteristical signal, δ | | | | | |

**BIOLOGICAL EXAMPLES**

Example No. 1

**Myzus persicae - test; (spray application)**

| | |
|---|---|
| Solvent: | 78 parts by weight acetone |
| | 1.5 parts by weight dimethylformamide |
| Wetting agent: | 0.5 parts by weight alkylarylpolyglcolether |

To produce a suitable preparation of active compound, 1 part by weight of active compound is mixed with the stated amount of solvent and emulsifier, and the concentrate is diluted with emulsifier-containing water to the desired concentration.

Chinese cabbage *(Brassica pekinesis)* leaf-disks infected with all instars of the green peach aphid *(Myzus persicae),* are sprayed with a preparation of the active ingredient at the desired concentration.

After the specified period of time, mortality in % is determined. 100% means that all aphids have been killed; 0% means that none of the aphids have been killed.

In this test for example, the following compounds from the preparation examples showed activity of ≥ 80 % at an application rate of 500 g/ha:

Example nos. 1-17, 1-4, 1-2, 1-14, 4-2, 3-1

Example No.2

**Myzus-Test; oral (spray application)**

Solvent: 80 parts by weight acetone

To produce a suitable preparation of active compound, 1 part by weight of active compound ist mixed with the stated amount of solvent and emulsifier, and the concentrate is diluted with emulsifier-containing water to the desired concentration.

Containers are infested with a mixed population of the green peach aphid *(Myzus persicae),* the aphids suck on a preparation of the active compound at the desired concentration.

After the specified period of time, mortality in % is determined. 100 % means that all aphids have been killed; 0 % means that none of the aphids have been killed.

In this test for example, the following compounds from the preparation examples showed activity of ≥ 80 % at an application rate of 100 ppm:

Example nos. 1-17, 1-4, 1-2

Example No.3

**Tetranychus urticae - test; OP-resistant (spray application)**

| | |
|---|---|
| Solvent: | 78 parts by weight acetone |
| | 1,5 parts by weight dimethylformamide |
| Wetting agent: | 0,5 parts by weight alkylarylpolyglcolether |

To produce a suitable preparation of active compound, 1 part by weight of active compound is mixed with the stated amount of solvent and emulsifier, and the concentrate is diluted with emulsifier-containing water to the desired concentration.

French bean *(Phaseolus vulgaris)* which are heavily infested with all stages of the two spotted spidermite *(Tetranychus urticae),* are sprayed with a preparation of the active ingredient at the desired concentration.

After the specified period of time, mortality in % is determined. 100% means that all spider mites have been killed and 0% means that none of the spider mites have been killed.

In this test for example, the following compounds from the preparation examples showed activity of ≥ 80 % at an application rate of 100 g/ha:

Example no. 1-2

Example No.4

**Meloidogyne test (MELGIN spray application)**

Solvent: 80 parts by weight of acetone

To produce a suitable preparation of active compound, 1 part by weight of active compound is mixed with the stated amount of solvent, and the concentrate is diluted with emulsifier-containing water to the desired concentration.

Vessels are filled with sand, a solution of the active ingredient, a suspension coutaining eggs and larvae of *Meloidogyne incognita* and salat seeds. The salat seeds germinate and the seedlings grow. Galls develop in the roots

After the specified period the nematicidal activity is determined on the basis of the percentage of gall formation. 100 % means that no galls were found; 0 % means that the number of galls found on the roots of treated plants was equal to that in untreated control plants.

In this test for example, the following compounds from the preparation examples showed activity of ≥ 80 % at an application rate of 20 ppm:

Example no. 1-2

Example No.5

**Myzus persicae - test**

Solvent: 7 parts by weight of dimethylformamide
Emulsifier: 2 part by weight of alkylaryl polyglycolether

To produce a suitable preparation of active compound, 1 part by weight of active compound is mixed with the stated amount of solvent and emulsifier, and the concentrate is diluted with emulsifier-containing water to the desired concentration.
Cabbage plants *(Brassica oleracea)* which are heavily infested by the green peach aphid *(Myzus persicae)* are treated by being drenched with the preparation of the active compound of the desired concentration.
After the specified period of time, mortality in % is determined. 100 % means that all the aphids have been killed; 0 % means that none of the aphids have been killed.
In this test for example, the following compounds from the preparation examples showed activity of ≥ 80 % at an application rate of 20 ppm:

Example no. 1-2

Example No.6

**Aphis gossypii - test**

Solvent: 7 parts by weight of dimethylformamide
Emulsifier: 2 parts by weight of alkylaryl polyglycolether

To produce a suitable preparation of active compound, 1 part by weight of active compound is mixed with the stated amount of solvent and emulsifier, and the concentrate is diluted with emulsifier-containing water to the desired concentration.
Cotton leaves *(Gossypium hirsutum)* which are heavily infested by the cotton aphid *(Aphis gossypii)* are treated by being dipped into the preparation of the active compound of the desired concentration.
After the specified period of time, the mortality in % is determined. 100 % means that all the aphids have been killed; 0 % means that none of the aphids have been killed.
In this test for example, the following compounds from the preparation examples showed activity of ≥ 80 % at an application rate of 20 ppm:

Example nos. 1-2, 1-14, 4-2

Example No.7

**Boophilus microplus - test (injektion)**

Solvent: dimethyl sulfoxide

To produce a suitable preparation of active compound, 10 mg of active compound are dissolved in 0.5 ml solvent, and the concentrate is diluted with water to the desired concentration.
Five adult engorged female ticks *(Boophilus microplus)* are injected with compound solution into the abdomen. Ticks are transferred into replica plates and incubated in a climate chamber for a period of time.
After the specified period of time, mortality in % is determined. 100 % means that all eggs are infertile; 0 % means that all eggs are fertile.
In this test for example, the following compounds from the preparation examples showed activity of ≥ 80 % at an application rate of 20μg/animal:
Example no. 1-2

## Claims

**1.** Compounds of the formula (I)

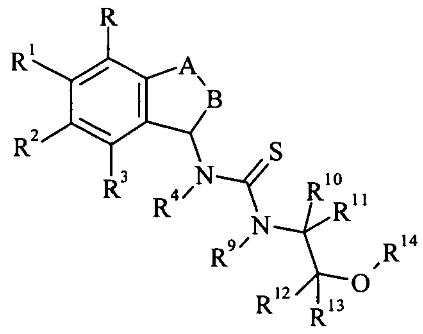

(I)

wherein

A is selected from $-CHR^5-$, $-CHR^5CHR^6-$, -O-, $-S(O)_n-$, $-NR^7-$ $-OCHR^5-$, - $CHR^5O-$, $-S(O)_nCHR^5-$, $-CHR^5S(O)_n-$, -C(X)- or $-C(=NOR^7)-$;

B is selected from $-CHR^8-$, -O- or $-S(O)_n-$;

X is selected from oxygen or sulphur;

n is an integer selected from 0, 1 or 2;

R, $R^1$, $R^2$ and $R^3$ are independently selected from hydrogen, halogen, hydroxy, alkyl, haloalkyl, alkoxy, haloalkoxy, alkylthio, haloalkylthio, alkenyl, haloalkenyl, alkynyl, cyano, nitro, optionally substituted aryl, optionally substituted aryloxy, optionally substituted heteroaryl, optionally substituted heteroaryloxy, alkoxyalkyl, alkoxyalkoxyalkyl, cycloalkyl, cyanoalkyl, formyl, alkoxycarbonyl, alkylcarbonyl, alkenylcarbonyl, dialkylphosphonato, amino, monoalkylamino, dialkylamino, cycloalkylamino, (cycloalkyl)(alkyl)amino, alkylsulfonyl, haloalkylsulfonyl, alkylsulfinyl, haloalkylsulfinyl, alkylsulfinylalkyl, alkylsulfonylalkyl, alkylthiocarbonyl and hydroxycarbonyl;

$R^4$ is selected from hydrogen, amino, hydroxyl, cyano, alkyl, haloalkyl, cycloalkyl, optionally substituted arylalkyl, alkenyl, alkynyl, cycloalkylalkyl, alkoxy, hydroxyalkyl, alkylsulfonyl, $-C(X)R^{15}$, $-C(X)OR^{16}$, $-C(X)SR^{16}$, - $S(O)_nR^{16}$, $-C(X)NR^{17}R^{18}$, $-S(O)_nNR^{17}R^{18}$, $-P(X)(OR^{19})(OR^{20})$, - $P(X)(NR^{21}R^{22})(NR^{23}R^{24})$, $-CHR^{25}-X-R^{26}$, $-CH=NR^{27}$ and $-C(=NR^{31})(SR^{32})$;

$R^5$ is selected from hydrogen, halogen, hydroxy, alkyl, alkoxy, alkylthio, haloalkyl, haloalkoxy, cyano, nitro and optionally substituted aryl;

$R^6$ is selected from hydrogen, halogen, hydroxy, alkyl, alkoxy, alkylthio, haloalkyl, haloalkoxy, cyano, nitro and optionally substituted aryl;

$R^7$ is selected from hydrogen or alkyl;

$R^8$ is selected from hydrogen, halogen, alkyl, alkoxy or optionally substituted aryl;

$R^9$ is selected from hydrogen, amino, hydroxyl, alkyl, haloalkyl, alkoxyalkyl, cycloalkyl, optionally substituted arylalkyl, alkenyl, alkynyl, cycloalkylalkyl, alkylcarbonyl, alkoxycarbonyl, alkylamino(thio)carbonyl, dialkylamino (thio)carbonyl, alkoxy, hydroxyalkyl;

$R^{10}$, $R^{11}$, $R^{12}$ and $R^{13}$ are independently selected from hydrogen, alkyl, haloalkyl, - alkoxy, alkoxyalkyl, alkenyl, cycloalkyl, cycloalkylalkyl, optionally substituted aryl, optionally substituted arylalkyl, optionally substituted hetaryl and optionally substituted hetarylalkyl;

$R^{14}$ is selected from hydrogen, alkyl, haloalkyl, alkoxyalkyl, hydroxyalkyl, cycloalkyl, optionally substituted arylalkyl, alkenyl, alkynyl, cycloalkylalkyl, alkylcarbonyl, alkoxycarbonyl, alkylamino(thio)carbonyl, dialkylamino (thio)carbonyl, optionally substituted arylcarbonyl and optionally substituted aryloxycarbonyl,

$R^{15}$ is selected from hydrogen and alkyl;

$R^{16}$ is selected from alkyl, haloalkyl, hydroxyalkyl, hydroxycarbonylalkyl and alkoxycarbonylalkyl;

$R^{17}$ and $R^{18}$ are independently selected from hydrogen, alkyl, haloalkyl, cycloalkyl, cyanoalkyl, optionally substituted aryl, alkylcarbonyl, alkoxycarbonyl, alkoxycarbonylalkyl, alkoxyalkyl, alkylthioalkyl, alkylsulfinylalkyl,

alkylsulfonylalkyl, mono or dialkylaminocarbonylalkyl, aminoalkyl, mono or dialkylaminoalkyl and optionally substituted arylcarbonyl;
$R^{19}$ and $R^{20}$ are independently selected from hydrogen, alkyl and haloalkyl;
$R^{21}$, $R^{22}$, $R^{23}$ and $R^{24}$ are independently selected from hydrogen and alkyl;
$R^{25}$ is selected from hydrogen and alkyl;
$R^{26}$ is selected from alkyl, haloalkyl, -C(X)$R^{28}$ and -P(X)(O$R^{29}$)(O$R^{30}$);
$R^{27}$ is selected from hydrogen, alkyl, haloalkyl, hydroxy, alkoxy, haloalkoxy and cyano;
$R^{28}$ is selected from hydrogen, alkyl and haloalkyl;
$R^{29}$ and $R^{30}$ are independently selected from hydrogen, alkyl and haloalkyl;
$R^{31}$ and $R^{32}$ are independently selected from hydrogen, alkyl, arylalkyl, -C(X)$R^{15}$, -$CO_2R^{16}$, $S(O)_nR^{16}$, -C(X)N$R^{17}R^{18}$, -$S(O)_n$N$R^{17}R^{18}$, -P(X)(O$R^{19}$)(O$R^{20}$), -P(X)(N$R^{21}R^{22}$)(N$R^{23}R^{24}$) and -CH$R^{25}$-X-$R^{26}$;
and
agriculturally acceptable salts thereof.

**2.** (S)-Enantiomers of the compounds of formula (I) according to Claim 1, in which

A is selected from -CH$R^5$-, -CH$R^5$CH$R^6$-, -OCH$R^5$-, -$S(O)_n$CH$R^5$-, -C(X)- or - C(=NO$R^7$)- and
B is selected from -CH$R^8$-.

**3.** (R)-Enantiomers of the compounds of formula (I) according to Claim 1, in which

A is selected from -CH$R^5$- -CH$R^5$CH$R^6$-, -O-, -$S(O)_n$-, -N$R^7$-, -OCH$R^5$-, - CH$R^5$O-, -$S(O)_n$CH$R^5$-, -CH$R^5$$S(O)_n$-, -C(X)- or -C(=NO$R^7$)- and
B is selected from -O- or -$S(O)_n$-.

**4.** (R)-Enantiomers of the compounds of formula (I) according to Claim 1, in which

A is selected from -O-, -$S(O)_n$-, -N$R^7$-, -CH$R^5$O-, -CH$R^5$$S(O)_n$- and
B is selected from -CH$R^8$-.

**5.** Chiral compounds of formula (II-a):

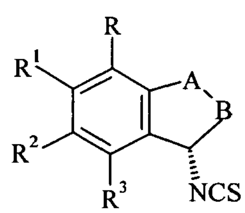

(II-a)

in which
A, B, R, $R^1$, $R^2$ and $R^3$ are as defined above, with the proviso that one of $R^1$ to $R^4$ is different from hydrogen or one of A and B is different from $CH_2$,

**6.** Composition comprising at least one compound of the formula (I) according to any of claims 1 to 4 and customary extenders and/or surfactants.

**7.** Method for controlling pests, wherein a compound of the formula (I) according to any of claims 1 to 4 or a composition according to claim 6 is allowed to act on the pests and/or their habitat.

**8.** Use of compounds of the formula (I) according to any of claims 1 to 4 or of compositions according to claim 6 for controlling pests.

European Patent Office

# EUROPEAN SEARCH REPORT

Application Number
EP 07 00 6900

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| E | WO 2007/060120 A (BASF AG [DE]; KORDES MARKUS [DE]; KORADIN CHRISTOPHER [DE]; CULBERTSON) 31 May 2007 (2007-05-31)<br>* abstract *<br>* page 17 - page 18; compounds IIIA-IIIC *<br>* table 1 *<br>* page 56 - page 57 * | 1,2,6-8 | INV.<br>A01N47/32<br>A01N47/36<br>C07C331/26<br>C07C335/14<br>C07D307/82<br>C07D311/68<br>C07D335/06<br>A01P7/02<br>A01P7/04 |
| X | WO 95/29165 A (SYNTEX INC [US])<br>2 November 1995 (1995-11-02)<br>* page 19; figure 2 *<br>* examples 10,11 * | 1,2,5,6 | |
| X,D | WO 2007/020377 A (SYNGENTA PARTICIPATIONS AG [CH]; SYNGENTA LTD [GB]; O'SULLIVAN ANTHONY) 22 February 2007 (2007-02-22)<br>* page 135; compound VII *<br>* example 11 * | 1,6 | |
| X | CAMBIE R C ET AL: "vic-Iodothiocyanates and Iodoisothiocyanates. Part 2. New Syntheses of Thiazolidin-2-ones and 2-Amino-2-thiazolines"<br>JOURNAL OF THE CHEMICAL SOCIETY, PERKIN TRANSACTIONS 1, CHEMICAL SOCIETY. LETCHWORTH, GB, no. 3, 1979, pages 765-770, XP003011657<br>ISSN: 0300-922X<br>* compound 11 * | 5 | TECHNICAL FIELDS SEARCHED (IPC)<br>A01N<br>C07C<br>C07D |
| A,D | WO 2006/125745 A (BASF AG [DE]; KORDES MARKUS [DE]; HOFMANN MICHAEL [DE]; PUHL MICHAEL []) 30 November 2006 (2006-11-30)<br>* abstract * | 1-8 | |

The present search report has been drawn up for all claims

1

| Place of search | Date of completion of the search | Examiner |
|---|---|---|
| Munich | 7 September 2007 | Molina de Alba, José |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 00 6900

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2007

| Patent document cited in search report | Publication date | Patent family member(s) | Publication date |
|---|---|---|---|
| WO 2007060120 A | 31-05-2007 | NONE | |
| WO 9529165 A | 02-11-1995 | AT 243199 T | 15-07-2003 |
| | | AU 687192 B2 | 19-02-1998 |
| | | AU 2294795 A | 16-11-1995 |
| | | AU 4099497 A | 08-01-1998 |
| | | BR 9507517 A | 16-09-1997 |
| | | CA 2188748 A1 | 02-11-1995 |
| | | CN 1151735 A | 11-06-1997 |
| | | CZ 9603109 A3 | 11-06-1997 |
| | | DE 69531102 D1 | 24-07-2003 |
| | | DE 69531102 T2 | 29-04-2004 |
| | | DK 757677 T3 | 13-10-2003 |
| | | EP 0757677 A1 | 12-02-1997 |
| | | ES 2201104 T3 | 16-03-2004 |
| | | FI 964312 A | 20-12-1996 |
| | | HU 76295 A2 | 28-07-1997 |
| | | IL 113480 A | 29-06-2000 |
| | | JP 9512269 T | 09-12-1997 |
| | | NO 964552 A | 27-12-1996 |
| | | NZ 284356 A | 19-12-1997 |
| | | PL 316856 A1 | 17-02-1997 |
| | | PT 757677 T | 28-11-2003 |
| | | RU 2145321 C1 | 10-02-2000 |
| WO 2007020377 A | 22-02-2007 | NONE | |
| WO 2006125745 A | 30-11-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2006125745 A
- WO 2007020377 A
- US 200604019 B
- US 2425615 A
- EP 538314 A
- US 5538988 A
- JP 37015645 B
- DE 2505301
- US 4005140 A

### Non-patent literature cited in the description

- *J. Org. Chem.,* 2006, vol. 71 (18), 6859
- *Chem. Eur. J.,* 2000, vol. 6 (10), 1840
- *J. Org. Chem.,* 2006, vol. 71 (18
- *Synthetic Communications,* 1992, vol. 22 (18), 2703-2710
- *Journal of Chemical Research, Synopses,* 1991, vol. 8, 201
- *Chemische Berichte,* 1964, vol. 97 (2), 510-519
- *Journal of Carbohydrate Chemistry,* 1995, vol. 14 (8), 1133-1152
- **CZECH.** *Collection of Czechoslovak Chemical Communications,* 1986, vol. 51 (1), 112-117